# EUROPEAN PATENT APPLICATION

(11) **EP 4 552 648 A1**
(43) Date of publication of application: **14.05.2025**
(21) Application number: 23835453.4
(22) Date of filing: 03.07.2023
(51) Int. Cl.: A61K 45/06, A61K 31/4184, A61K 31/4427, A61K 31/4523, A61K 31/496, A61K 31/498, A61K 31/506, A61K 31/517, A61K 31/519, A61K 31/537, A61K 31/5377, A61K 31/66, A61P 35/00, A61P 43/00

(54) **COMBINATION DRUG**

(30) Priority: 04.07.2022 JP 2022107937; 01.11.2022 JP 2022175601
(71) Applicant: CHUGAI SEIYAKU KABUSHIKI KAISHA, Tokyo 115-8543 (JP)
(72) Inventor: FURUGAKI Ko, Kamakura-shi, Kanagawa 247-8530 (JP)
(74) Representative: Vossius & Partner Patentanwälte Rechtsanwälte mbB
(86) International application number: PCT/JP2023/024539
(87) International publication number: WO 2024/009921

(57) **Abstract**

The present invention relates to a drug for treating or preventing ALK fusion gene-positive cancer positive for FGFR1 expression and positive for FGF2 expression at baseline, the drug comprising a compound having ALK inhibitory activity and a FGFR inhibitor in combination, a combination agent, a pharmaceutical composition, a preparation, a treatment or prevention method, or a drug for suppressing acquisition of resistance, a method for selecting a patient, and the like.

## Description

### [Technical Field]

The present invention relates to a drug, a combination agent, a pharmaceutical composition or a preparation which is useful for the treatment or prevention of ALK fusion gene-positive cancer positive for FGFR1 expression and positive for FGF2 expression at baseline, and comprises an ALK inhibitor and a FGFR kinase inhibitor in combination, as well as a method and a product for treating or preventing target cancer, a method for selecting a patient with ALK fusion gene-positive cancer positive for FGFR1 expression and positive for FGF2 expression at baseline, and the like.

### [Background Art]

Anaplastic lymphoma kinase (ALK) is a proto-oncogene of a receptor tyrosine kinase which was identified as a fusion gene in 1994 (Non Patent Literature 1).

It has been reported that genetic abnormalities (translocations) in ALK result in the production of abnormal kinases, which are involved in canceration (Science. 1994 Mar 4; 263(5151): 1281-4.: Non Patent Literature 1). For example, it has been reported that, in lung cancer, ALK is fused to the microtubule associated protein EML4 through chromosomal translocation, and generates EML4-ALK which has active tyrosine kinase activity, thereby gaining oncogenic potential (Cancer Res. 2008 Jul 1; 68(13): 4971-6.: Non Patent Literature 2). It has been reported that compounds having ALK kinase inhibitory activity are useful for the cancers with these genetic abnormalities (Non Patent Literature 2).

Alectinib (generic name: alectinib hydrochloride) is approved in Japan and overseas as an ALK inhibitor.

The fibroblast growth factor receptor (FGFR) is a receptor tyrosine kinase, and it has been reported that genetic abnormalities (mutations and translocations) result in production of abnormal kinases, which are involved in canceration (Wiley Interdiscip Rev Dev Biol. May-Jun 2015; 4(3): 215-66.: Non Patent Literature 4). FGFR inhibitors are known to suppress the phosphorylation of the FGFR kinase, thereby suppressing the growth of cancer cells (Nat Med.2018 May; 24(4): 512-517: Non Patent Literature 5).

On the other hand, such inhibitors have been known to have a problem that although immediately after the start of the treatment, an dramatic effect is exhibited against cancer to be treated with the inhibitors, the effect diminishes as cancer cells acquire resistance through long-term administration (Cell 2010.141(1): 69-8: Non Patent Literature 3).

As a mechanism of acquisition of resistance by cancer cells under other drugs, the involvement of FGF2 and FGFR1 in resistance to an EGFR inhibitor (gefitinib) has been reported (Oncogenesis. 2013 Mar; 2(3): e39: Non Patent Literature 6).

Suppression of cell growth by combined use of different inhibitors against cancer cells that have acquired resistance through inhibitor treatment is examined. For example, an effect of combined use of ceritinib as an ALK inhibitor and a FGFR inhibitor (infigratinib) on a resistant strain established by exposing ceritinib to an ALK lung cancer cell strain derived from an ALK lung cancer patient has been reported (Non Patent Literature 5).

However, it has been considered that the involvement of FGFR is not a major part of the mechanism of acquisition of EGFR inhibitor resistance (Annals of Oncology 29 (Supplement 1): i10-i19, 2018: Non Patent Literature 7), and for the mechanism of acquisition of resistance under an ALK inhibitor, G1202R mutations, I1171N mutations and the like caused by a change in three-dimensional structure of ALK fusion protein are major resistance mechanisms (Frontiers in Oncology. 2021 October; Vol. 11, 1-13: Non Patent Literature 8, Journal of Clinical Oncology 37, no. 16 (June 01, 2019) 1370-1379: Non Patent Literature 9).

### [Citation List]

[Non Patent Literature 1] Science. 1994 Mar 4; 263(5151): 1281-4.
[Non Patent Literature 2] Cancer Res. 2008 Jul 1;68(13):4971-6.
[Non Patent Literature 3] Cell 2010.141(1): 69-8
[Non Patent Literature 4] Wiley Interdiscip Rev Dev Biol. May-Jun 2015;4(3):215-66.
[Non Patent Literature 5] Nat Med.2018 May;24(4):512-517
[Non Patent Literature 6] Oncogenesis. 2013 Mar; 2(3): e39
[Non Patent Literature 7] Annals of Oncology 29 (Supplement 1): i10-i19, 2018
[Non Patent Literature 8] Frontiers in Oncology. 2021 October; Vol. 11, 1-13
[Non Patent Literature 9] Journal of Clinical Oncology 37, no. 16 (June 01, 2019) 1370-1379

### [Summary of Invention]

In the situations described above, it is desired to provide a therapeutic agent which is effective for ALK fusion gene-positive cancer that has acquired resistance, and/or capable of suppressing the acquisition of resistance by ALK fusion gene-positive cancer.

With attention given to heretofore unknown associations between ALK fusion gene-positive cancer and two cancer genes, i.e., FGFR1 and FGF2, the present invention provides a novel therapy comprising administering an ALK inhibitor in combination with a FGFR kinase inhibitor to ALK fusion gene-positive cancer having a combination of the cancer genes.

That is, the present invention provides a therapeutic agent having an excellent growth suppressive effect on ALK fusion gene-positive cancer positive for FGFR1 expression and positive for FGF2 expression at baseline by identifying a gene involved in the mechanism of acquisition of resistance by ALK fusion gene-positive cancer cells under an ALK inhibitor, and using a compound having ALK inhibitory activity in combination with a FGFR inhibitor.

Another aspect of the present invention provides an agent for suppressing the acquisition of resistance by ALK fusion gene-positive cancer positive for FGFR1 expression and positive for FGF2 expression at baseline by using a compound having ALK inhibitory activity in combination with a FGFR inhibitor on the cancer before acquisition of resistance.

That is, the present invention relates to the following invention.
<1> A drug for treating or preventing ALK fusion gene-positive cancer positive for FGFR1 expression and positive for FGF2 expression at baseline, the drug comprising an ALK inhibitor and a FGFR inhibitor in combination.
   <1-a1> The drug according to <1>, wherein the ALK inhibitor is a compound selected from crizotinib, alectinib, ceritinib, lorlatinib and brigatinib, or a salt thereof.
   <1-a2> The drug according to <1>, wherein the ALK inhibitor is a compound selected from alectinib and lorlatinib, or a salt thereof.
   <1-b1> The drug according to any one of <1> to <1-a2>, wherein the FGFR inhibitor is a compound selected from the group consisting of infigratinib, derazantinib, pemigatinib, erdafitinib, ponatinib, Debio 1347, futibatinib, tasurgratinib, AZD 4547 and PD 173074, or a salt thereof.
   <1-b2> The drug according to any one of <1> to <1-a2>, wherein the FGFR inhibitor is AZD 4547 or infigratinib.
   <1-c1> The drug according to any one of <1> to <1-b2>, wherein the ALK fusion gene-positive cancer is cancer selected from the group consisting of non-Hodgkin's lymphoma, neuroblastoma, inflammatory myofibroblastic cytoma, kidney cancer and lung cancer.
   <1-c2> The drug according to any one of <1> to <1-b2>, wherein the ALK fusion gene-positive cancer is non-small cell lung cancer.
   <1-d> The drug according to any one of <1> to <1-b2>, wherein the ALK fusion gene-positive cancer has not received prior treatment with an ALK inhibitor.
   <1-e> The drug according to any one of <1> to <1-d>, wherein the ALK fusion gene-positive cancer is sensitive to ALK.
   <1-f> The drug according to any one of <1> to <1-e>, wherein the ALK inhibitor is administered simultaneously with the FGFR inhibitor.
<2> A drug for suppressing the acquisition of resistance by ALK fusion gene-positive cancer positive for FGFR1 expression and positive for FGF2 expression at baseline, the drug comprising an ALK inhibitor and a FGFR inhibitor in combination.
   <2-a1> The drug according to <2>, wherein the ALK inhibitor is a compound selected from crizotinib, alectinib, ceritinib, lorlatinib and brigatinib, or a salt thereof.
   <2-a2> The drug according to <2>, wherein the ALK inhibitor is a compound selected from alectinib and lorlatinib, or a salt thereof.
   <2-b1> The drug according to any one of <2> to <2-a2>, wherein the FGFR inhibitor is a compound selected from the group consisting of infigratinib, derazantinib, pemigatinib, erdafitinib, ponatinib, Debio 1347, futibatinib, tasurgratinib, AZD 4547 and PD 173074, or a salt thereof.
   <2-b2> The drug according to any one of <2> to <2-a2>, wherein the FGFR inhibitor is AZD 4547 or infigratinib.
   <2-c1> The drug according to any one of <2> to <2-b2>, wherein the ALK fusion gene-positive cancer is cancer selected from the group consisting of non-Hodgkin's lymphoma, neuroblastoma, inflammatory myofibroblastic cytoma, kidney cancer and lung cancer.
   <2-c2> The drug according to any one of <2> to <2-b2>, wherein the ALK fusion gene-positive cancer is non-small cell lung cancer.
   <2-d> The drug according to any one of <2> to <2-b2>, wherein the ALK fusion gene-positive cancer has not received prior treatment with an ALK inhibitor.
   <2-e> The drug according to any one of <2> to <2-d>, wherein the ALK fusion gene-positive cancer is sensitive to ALK.
   <2-f> The drug according to any one of <2> to <2-e>, wherein the ALK inhibitor is administered simultaneously with the FGFR inhibitor.
<3> A drug for treating or preventing ALK fusion gene-positive cancer positive for FGFR1 expression and positive for FGF2 expression at baseline, the drug comprising an ALK inhibitor as an active ingredient, which is used in combination with a FGFR inhibitor.
   <3-a1> The drug according to <3>, wherein the ALK inhibitor is a compound selected from crizotinib, alectinib, ceritinib, lorlatinib and brigatinib, or a salt thereof.
   <3-a2> The drug according to <3>, wherein the ALK inhibitor is a compound selected from alectinib and lorlatinib, or a salt thereof.
   <3-b1> The drug according to any one of <3> to <3-a2>, wherein the FGFR inhibitor is a compound selected from the group consisting of infigratinib, derazantinib, pemigatinib, erdafitinib, ponatinib, Debio 1347, futibatinib, tasurgratinib, AZD 4547 and PD 173074, or a salt thereof.
   <3-b2> The drug according to any one of <3> to <3-a2>, wherein the FGFR inhibitor is AZD 4547 or infigratinib.
   <3-c1> The drug according to any one of <3> to <3-b2>, wherein the ALK fusion gene positive cancer is cancer selected from the group consisting of non-Hodgkin's lymphoma, neuroblastoma, inflammatory myofibroblastic cytoma, kidney cancer and lung cancer.
   <3-c2> The drug according to any one of <3> to <3-b2>, wherein the ALK fusion gene positive cancer is non-small cell lung cancer.
   <3-d> The drug according to any one of <3> to <3-b2>, wherein the ALK fusion gene positive cancer has not received prior treatment with an ALK inhibitor.
   <3-e> The drug according to any one of <3> to <3-d>, wherein the ALK fusion gene positive cancer is sensitive to ALK.
   <3-f> The drug according to any one of <3> to <3-e>, wherein the ALK inhibitor is administered simultaneously with the FGFR inhibitor.
<4> A drug for suppressing the acquisition of resistance by ALK fusion gene-positive cancer positive for FGFR1 expression and positive for FGF2 expression at baseline, the drug comprising an ALK inhibitor as an active ingredient, which is used in combination with a FGFR inhibitor.
   <4-a1> The drug according to <4>, wherein the ALK inhibitor is a compound selected from crizotinib, alectinib, ceritinib, lorlatinib and brigatinib, or a salt thereof.
   <4-a2> The drug according to <4>, wherein the ALK inhibitor is a compound selected from alectinib and lorlatinib, or a salt thereof.
   <4-b1> The drug according to any one of <4> to <4-a2>, wherein the FGFR inhibitor is a compound selected from the group consisting of infigratinib, derazantinib, pemigatinib, erdafitinib, ponatinib, Debio 1347, futibatinib, tasurgratinib, AZD 4547 and PD 173074, or a salt thereof.
   <4-b2> The drug according to any one of <4> to <4-a2>, wherein the FGFR inhibitor is AZD 4547 or infigratinib.
   <4-c1> The drug according to any one of <4> to <4-b2>, wherein the ALK fusion gene-positive cancer is cancer selected from the group consisting of non-Hodgkin's lymphoma, neuroblastoma, inflammatory myofibroblastic cytoma, kidney cancer and lung cancer.
   <4-c2> The drug according to any one of <4> to <4-b2>, wherein the ALK fusion gene-positive cancer is non-small cell lung cancer.
   <4-d> The drug according to any one of <4> to <4-c2>, wherein the ALK fusion gene-positive cancer has not received prior treatment with an ALK inhibitor.
   <4-e> The drug according to any one of <4> to <4-d>, wherein the ALK fusion gene-positive cancer is sensitive to ALK.
   <4-f> The drug according to any one of <4> to <4-e>, wherein the ALK inhibitor is administered simultaneously with the FGFR inhibitor.
<5> A drug for treating or preventing ALK fusion gene-positive cancer positive for FGFR1 expression and positive for FGF2 expression at baseline, the drug comprising a FGFR inhibitor as an active ingredient, which is used in combination with an ALK inhibitor.
   <5-a1> The drug according to <5>, wherein the ALK inhibitor is a compound selected from crizotinib, alectinib, ceritinib, lorlatinib and brigatinib, or a salt thereof.
   <5-a2> The drug according to <5>, wherein the ALK inhibitor is a compound selected from alectinib and lorlatinib, or a salt thereof.
   <5-b1> The drug according to any one of <5> to <5-a2>, wherein the FGFR inhibitor is a compound selected from the group consisting of infigratinib, derazantinib, pemigatinib, erdafitinib, ponatinib, Debio 1347, futibatinib, tasurgratinib, AZD 4547 and PD 173074, or a salt thereof.
   <5-b2> The drug according to any one of <5> to <5-a2>, wherein the FGFR inhibitor is AZD 4547 or infigratinib.
   <5-c1> The drug according to any one of <5> to <5-b2>, wherein the ALK fusion gene-positive cancer is cancer selected from the group consisting of non-Hodgkin's lymphoma, neuroblastoma, inflammatory myofibroblastic cytoma, kidney cancer and lung cancer.
   <5-c2> The drug according to any one of <5> to <5-b2>, wherein the ALK fusion gene-positive cancer is non-small cell lung cancer.
   <5-d> The drug according to any one of <5> to <5-c2>, wherein the ALK fusion gene-positive cancer has not received prior treatment with an ALK inhibitor.
   <5-e> The drug according to any one of <5> to <5-d>, wherein the ALK fusion gene-positive cancer is sensitive to ALK.
   s<5-f> The drug according to any one of <5> to <5-e>, wherein the FGFR inhibitor is administered simultaneously with the ALK inhibitor.
<6> A drug for suppressing the acquisition of resistance by ALK fusion gene-positive cancer positive for FGFR1 expression and positive for FGF2 expression at baseline, the drug comprising a FGFR inhibitor as an active ingredient, which is used in combination with an ALK inhibitor.
   <6-a1> The drug according to <6>, wherein the ALK inhibitor is a compound selected from crizotinib, alectinib, ceritinib, lorlatinib and brigatinib, or a salt thereof.
   <6-a2> The drug according to <6>, wherein the ALK inhibitor is a compound selected from alectinib and lorlatinib, or a salt thereof.
   <6-b1> The drug according to any one of <6> to <6-a2>, wherein the FGFR inhibitor is a compound selected from the group consisting of infigratinib, derazantinib, pemigatinib, erdafitinib, ponatinib, Debio 1347, futibatinib, tasurgratinib, AZD 4547 and PD 173074, or a salt thereof.
   <6-b2> The drug according to any one of <6> to <6-a2>, wherein the FGFR inhibitor is AZD 4547 or infigratinib.
   <6-c1> The drug according to any one of <6> to <6-b2>, wherein the ALK fusion gene-positive cancer is cancer selected from the group consisting of non-Hodgkin's lymphoma, neuroblastoma, inflammatory myofibroblastic cytoma, kidney cancer and lung cancer.
   <6-c2> The drug according to any one of <6> to <6-b2>, wherein the ALK fusion gene-positive cancer is non-small cell lung cancer.
   <6-d> The drug according to any one of <6> to <6-c2>, wherein the ALK fusion gene-positive cancer has not received prior treatment with an ALK inhibitor.
   <6-e> The drug according to any one of <6> to <6-d>, wherein the ALK fusion gene-positive cancer is sensitive to ALK.
   <6-f> The drug according to any one of <6> to <6-e>, wherein the FGFR inhibitor is administered simultaneously with the ALK inhibitor.
<7> A method for treating or preventing ALK fusion gene-positive cancer positive for FGFR1 expression and positive for FGF2 expression at baseline, the method comprising administrating to a subject an effective amount of a compound having ALK inhibitory activity and an effective amount of a FGFR inhibitor in combination.
   <7-a1> The drug according to <7>, wherein the ALK inhibitor is a compound selected from crizotinib, alectinib, ceritinib, lorlatinib and brigatinib, or a salt thereof.
   <7-a2> The method according to <7>, wherein the ALK inhibitor is a compound selected from alectinib and lorlatinib, or a salt thereof.
   <7-b1> The method according to any one of <7> to <7-a2>, wherein the FGFR inhibitor is a compound selected from the group consisting of infigratinib, derazantinib, pemigatinib, erdafitinib, ponatinib, Debio 1347, futibatinib, tasurgratinib, AZD 4547 and PD 173074, or a salt thereof.
   <7-b2> The method according to any one of <7> to <7-a2>, wherein the FGFR inhibitor is AZD 4547 or infigratinib.
   <7-c1> The method according to any one of <7> to <7-b2>, wherein the ALK fusion gene-positive cancer is cancer selected from the group consisting of non-Hodgkin's lymphoma, neuroblastoma, inflammatory myofibroblastic cytoma, kidney cancer and lung cancer.
   <7-c2> The method according to any one of <7> to <7-b2>, wherein the ALK fusion gene-positive cancer is non-small cell lung cancer.
   <7-d> The method according to any one of <7> to <7-c2>, wherein the ALK fusion gene-positive cancer has not received prior treatment with an ALK inhibitor.
   <7-e> The method according to any one of <7> to <7-d>, wherein the ALK fusion gene-positive cancer is sensitive to ALK.
   <7-f> The method according to any one of <7> to <7-e>, wherein the ALK inhibitor is administered simultaneously with the FGFR inhibitor.
<8> A method for suppressing the acquisition of resistance by ALK fusion gene-positive cancer positive for FGFR1 expression and positive for FGF2 expression at baseline, the method comprising administrating to a subject an effective amount of a compound having ALK inhibitory activity and an effective amount of a FGFR inhibitor in combination.
   <8-a1>, The drug according to <8>, wherein the ALK inhibitor is a compound selected from crizotinib, alectinib, ceritinib, lorlatinib and brigatinib, or a salt thereof.
   <8-a2> The method according to <8>, wherein the ALK inhibitor is a compound selected from alectinib and lorlatinib, or a salt thereof.
   <8-b1> The method according to any one of <8> to <8-a2>, wherein the FGFR inhibitor is a compound selected from the group consisting of infigratinib, derazantinib, pemigatinib, erdafitinib, ponatinib, Debio 1347, futibatinib, tasurgratinib, AZD 4547 and PD 173074, or a salt thereof.
   <8-b2> The method according to any one of <8> to <8-a2>, wherein the FGFR inhibitor is AZD 4547 or infigratinib.
   <8-c1> The method according to any one of <8> to <8-b2>, wherein the ALK fusion gene-positive cancer is cancer selected from the group consisting of non-Hodgkin's lymphoma, neuroblastoma, inflammatory myofibroblastic cytoma, kidney cancer and lung cancer.
   <8-c2> The method according to any one of <8> to <8-b2>, wherein the ALK fusion gene-positive cancer is non-small cell lung cancer.
   <8-d> The method according to any one of <8> to <8-c2>, wherein the ALK fusion gene-positive cancer has not received prior treatment with an ALK inhibitor.
   <8-e> The method according to any one of <8> to <8-d>, wherein the ALK fusion gene-positive cancer is sensitive to ALK.
   <8-f> The method according to any one of <8> to <8-e>, wherein the ALK inhibitor is administered simultaneously with the FGFR inhibitor.
<9> A product comprising: (1) a preparation comprising a compound having ALK kinase inhibitory activity, (2) a container, and (3) an instruction or label indicating that the compound having ALK inhibitory activity and at least one FGFR inhibitor are administered in combination to a subject for treating ALK fusion gene-positive cancer positive for FGFR1 expression and positive for FGF2 expression at baseline.
   <9-a1> The drug according to <9>, wherein the ALK inhibitor is a compound selected from crizotinib, alectinib, ceritinib, lorlatinib and brigatinib, or a salt thereof.
   <9-a2> The product according to <9>, wherein the ALK inhibitor is a compound selected from alectinib and lorlatinib, or a salt thereof.
   <9-b1> The product according to any one of <9> to <9-a2>, wherein the FGFR inhibitor is a compound selected from the group consisting of infigratinib, derazantinib, pemigatinib, erdafitinib, ponatinib, Debio 1347, futibatinib, tasurgratinib, AZD 4547 and PD 173074, or a salt thereof.
   <9-b2> The product according to any one of <9> to <9-a2>, wherein the FGFR inhibitor is AZD 4547 or infigratinib.
   <9-c1> The product according to any one of <9> to <9-b2>, wherein the ALK fusion gene-positive cancer is cancer selected from the group consisting of non-Hodgkin's lymphoma, neuroblastoma, inflammatory myofibroblastic cytoma, kidney cancer and lung cancer.
   <9-c2> The product according to any one of <9> to <9-b2>, wherein the ALK fusion gene-positive cancer is non-small cell lung cancer.
   <9-d> The product according to any one of <9> to <9-c2>, wherein the ALK fusion gene-positive cancer has not received prior treatment with an ALK inhibitor.
   <9-e> The product according to any one of <9> to <9-d>, wherein the ALK fusion gene-positive cancer is sensitive to ALK.
   <9-f> The product according to any one of <9> to <9-e>, wherein the ALK inhibitor is administered simultaneously with the FGFR inhibitor.
<10> A method for selecting a patient with ALK fusion gene-positive cancer positive for FGFR1 expression and positive for FGF2 expression at baseline, the method comprising acquiring a tumor tissue of a patient diagnosed as ALK fusion gene-positive cancer, and measuring expression levels of FGFR1 and FGF2 in the tumor tissue specimen.
   <10-a> The method according to <10>, wherein the expression level is an expression level of protein.
   <10-b> The method according to <10>, wherein the expression level is an expression level of mRNA.
   <10-c> The method according to any one of <10> to <10-c>, wherein the specimen is a fresh-frozen, fix-frozen or formalin-fixed paraffin-embedded (FFPE) specimen.

### [Brief Description of Drawings]

[Figure 1] Figure 1 shows graphs showing a cell growth suppressive effect of each inhibitor on cell growth in (A) untreated NCI-H2228 (parental cells), (B) DTP cells and (C) regrown cells.
[Figure 2] Figure 2 shows (A) a Western blotting showing the detection of ALK or FGFR1 signal molecules in untreated NCI-H2228 (parental cells) and DTP cells, and (B) a graph showing a change in the amount of phosphorylated FGFR1 in BGJ398 treatment in DTP cells.
[Figure 3] Figure 3 shows (A) a Western blotting showing a time-dependent change in ALK or FGFR1 signal molecules and (B) a graph showing a time-dependent change in the amount of phosphorylated FGFR1 through alectinib treatment in untreated NCI-H2228 (parental cells).
[Figure 4] Figure 4 shows photographs in which cell morphological changes in untreated NCI-H2228 (parental cells) and DTP cells are compared.

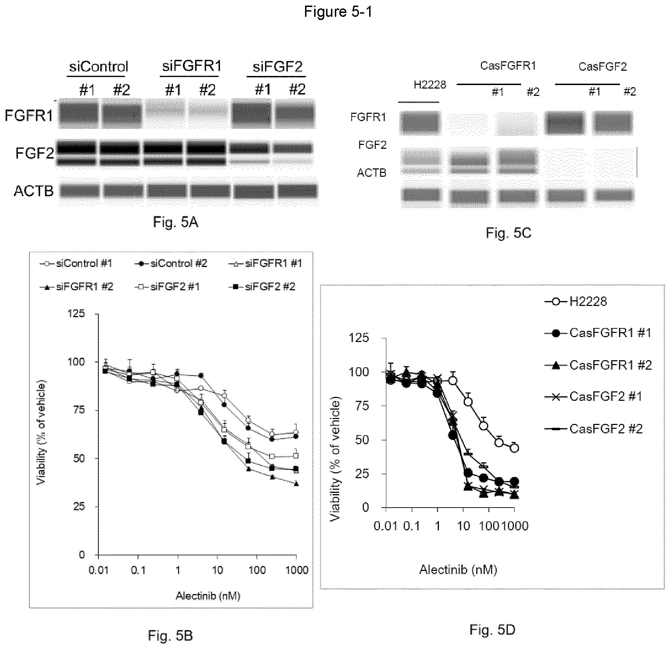

[Figure 5-1] Figure 5A shows a Western blotting showing the expression level of FGFR1 or the expression level of FGF2 in FGFR1 or FGF2 knockout cells with SiRNA. Figure 5B is a graph showing a cell growth suppressive effect of treatment with alectinib in FGFR1 or FGF2 knockout cells with the CRISPR/Cas9 system. Figure 5C shows a Western blotting showing the expression level of FGFR1 or the expression level of FGF2 in FGFR1 or FGF2 knockout cells with the CRISPR/Cas9 system. Figure 5D is a graph showing a cell growth suppressive effect of treatment with alectinib in FGFR1 or FGF2 knockout cells with the CRISPR/Cas9 system.
[Figure 5-2] Figure 5E is a graph showing a cell growth suppressive effect of treatment with lorlatinib in FGFR1 or FGF2 knockout cells with the CRISPR/Cas9 system. Figure 5F is a graph showing an inducible apoptosis-inducing effect of alectinib treatment in FGFR1 or FGF2 knockout cells. Figure 5G shows a Western blotting showing a change in signals downstream of FGFR1 through treatment with a control and alectinib in FGFR1 or FGF2 knockout cells with the CRISPR/Cas9 system.
[Figure 6] Figure 6 shows a Western blotting in which the amounts of FGFR1 and FGF2 proteins in SNU2535 parental cells and NCI-H2228 parental cells are compared.
[Figure 7] Figure 7 shows (A) a graph showing a cell growth suppressive effect of treatment with alectinib in SNU-2535 cells excessively expressing FGFR1 and FGF2, and (B) a Western blotting showing a change in ALK and FGFR1 signal molecules through alectinib treatment.
[Figure 8] Figure 8 is a graph showing a cell growth suppressive effect of treatment with alectinib and BGJ398 singly/in combination in alectinib-resistant NCI-H2228 cells.
[Figure 9] Figure 9 shows a Western blotting showing the detection of ALK and FGFR1 signal molecules in NCI-H2228 parental cells and alectinib-resistant cells.
[Figure 10] Figure 10 shows a Western blotting showing the detection of ALK and FGFR1 signal molecules through treatment with alectinib and BGJ398 singly/in combination in NCI-H2228 parental cells and alectinib-resistant cells.
[Figure 11] Figure 11 shows a graph showing a cell growth suppressive effect of treatment with alectinib and a FGFR inhibitor in combination in (A) NCI-H2228 and (C) SNU-2535 cells, and a graph showing a cell growth suppressive effect of treatment with lorlatinib and a FGFR inhibitor in combination in cell growth of (B) NCI-H2228 and (D) SNU-2535 cells.
[Figure 12] Figure 12 shows a Western blotting showing the detection of ALK and FGFR1 signal molecules through treatment of NCI-H2228 and SNU-2535 cells with alectinib and BGJ398 singly/in combination.
[Figure 13] Figure 13 shows a graph showing induction of apoptosis through treatment with alectinib and a FGFR inhibitor in combination in (A) NCI-H2228 and (B) SNU-2535 cells.
[Figure 14] Figure 14 shows (A) a graph showing a tumor growth suppressive effect, (B) a Western blotting showing the detection of ALK and FGFR1 signal molecules, (C) a photograph showing a tumor size, and (D) a graph showing a tumor weight, in administration of alectinib and GJ398 singly/in combination in NCI-H2228 tumor-transplanted mouse models.
[Figure 15] Figure 15 is a graph showing a change in body weight in single/combined administration of alectinib and BGJ398 in NCI-H2228 tumor-transplanted mouse models.
[Figure 16] Figure 16 is a graph showing a cell growth suppressive effect of long-term treatment of NCI-H2228 cells with alectinib and BGJ398 singly/in combination.
[Figure 17] Figure 17 is a graph showing a mRNA expression level of FGFR1 versus a hazard ratio.
[Figure 18] Figure 18 is a graph showing a mRNA expression level of FGF2 versus a hazard ratio.
[Figure 19] Figure 19 is a graph obtained by plotting a mRNA expression level of FGFR1 and a mRNA expression level of FGF2.

### [Description of Embodiments]

The present invention relates to a drug, a combination agent, a pharmaceutical composition, a preparation and a product for treating or preventing ALK fusion gene-positive cancer positive for FGFR1 expression and positive for FGF2 expression at baseline, which comprise a compound having ALK inhibitory activity and a FGFR inhibitor in combination. The present invention also relates to a method for treating or preventing ALK fusion gene-positive cancer positive for FGFR1 expression and positive for FGF2 expression at baseline, a drug for suppressing the acquisition of resistance by ALK fusion gene-positive cancer positive for FGFR1 expression and positive for FGF2 expression at baseline, or a method for suppressing the acquisition of resistance.

### ALK inhibitor

The ALK inhibitor for use in the present invention means a substance capable of directly or indirectly neutralizing, blocking, inhibiting, reducing, or preventing the ALK activity. Examples of the activity of ALK include tyrosine kinase activity. The substance includes a low molecular weight compound, an antibody, an antisense, a transcription inhibitor, a small interfering RNA (siRNA) and the like.

As examples of the low molecular weight compound, first-generation crizotinib (compound name: 3-[(1R-1-(2,6-dichloro-3-fluorophenyl)ethoxy]-5-[1-(piperidin-4-yl)-1H-pyrazol-4-yl]pyridin-2-amine), second-generation alectinib (compound name: 9-ethyl-6,6-dimethyl-8-[4-(morpholin-4-yl)-piperidin-1-yl]-11-oxo-6,11-dihydro-5H-benzo[b]carbazole-3-carbonitrile-hydrochloride), ceritinib (compound name: 5-chloro-N2-(2-isopropoxy-5-methyl-4-piperidin-4-yl-phenyl)-N4-[2-(propane-2-sulfonyl)-phenyl]-pyrimidine-2,4-diamine), and third-generation lorlatinib (compound name: (10R)-7-amino-12-fluoro-2,10,16-trimethyl-15-oxo-10,15,16,17-tetrahydro-2H-4,8-methanepyrazolo[4,3-h][2,5,11]benzoxadiazacyclotetradecine-3-carbonitrile) have been approved up to now, and brigatinib (compound name: {2-[(5-chloro-2-{ 2-methoxy-4-[4-(4-methylpiperazin-1-yl)piperidin-1-yl] anilino } pyrimidin-4-yl)amino]phenyl}dimethyl-λ⁵-phosphanone) has been approved as the fifth agent in January, 2021.

Alectinib is widely used as a drug of choice. These compounds or salts thereof can be produced according to the methods described in WO2010/143664, WO2004/076412, WO2006/021881, WO2008/073687 and the like. These compounds or salts thereof also include hydrates, various pharmaceutically acceptable solvates, crystalline polymorphs and the like.

As a route of administration of the ALK inhibitor for use in the present invention, either oral or parenteral administration is suitably used, but preferably, oral administration is suitably used. The dosage form used for oral administration may be appropriately selected from any dosage form such as a liquid, a powder, granules, a tablet, an enteric coated drug, and a capsule. The ALK inhibitor having such dosage forms is formulated by a method known to those skilled in the art. For example, the compound is formulated by appropriately combining it with a pharmaceutically acceptable carrier or medium, specifically, sterile water or saline, vegetable oil, an emulsifier, a suspending agent, a surfactant, a stabilizer, a flavoring agent, an excipient, a vehicle, a preservative, a binder, and the like, and mixing it in a unit dose form required for generally accepted clinical practices, then by formulating operations such as freeze-drying and tablet compression.

The ALK inhibitor may also be used parenterally in the form of an injection, such as a sterile solution or suspension with water or other pharmaceutically acceptable fluids. The amount of active ingredient in these preparations is selected as appropriate so that the appropriate dose within the indicated range may be administered. A sterile composition for injection may be formulated according to normal preparation practices using a vehicle such as distilled water for injection. Examples of aqueous solutions for injection include saline, an isotonic solution containing glucose or other adjuncts, for example, D-sorbitol, D-mannose, D-mannitol, and sodium chloride, which may be appropriately used in combination with an appropriate solubilizing agent, for example, an alcohol, specifically ethanol, a polyalcohol, for example, propylene glycol and polyethylene glycol, and a nonionic surfactant, for example, polysorbate 80 (TM) and HCO-50. Examples of oily liquids include sesame oil and soybean oil, which may be used in combination with benzyl benzoate and benzyl alcohol as a solubilizing agent. In addition, it may also be suitably blended with a buffering agent, for example, a phosphate buffer solution and a sodium acetate buffer solution, a soothing agent, for example, procaine hydrochloride, a stabilizer, for example, benzyl alcohol and phenol, and an antioxidant.

The dose of the ALK inhibitor according to the present invention may be selected, for example, within the range of 0.0001 mg to 1000 mg per kg of body weight per administration. Alternatively, for example, the dose may be selected within the range of 0.001 mg to 100,000 mg/body per patient. However, the dose of the ALK inhibitor of the present invention is not limited to these doses.

Examples of a more specific dose of the alectinib, the salt thereof, or the hydrate thereof include 20 mg, 40 mg, 60 mg, 80 mg, 120 mg, 160 mg, 220 mg, 240 mg, 300 mg, 460 mg, 600 mg, 760 mg, and 900 mg in free form per dose, twice daily.

The administration period of the ALK inhibitor of the present invention is appropriately determined according to the degree of symptoms and side effects, and can be administered until the cancer is treated or the desired therapeutic effect is achieved. Specifically, the administration may be continuously performed for 7 days to 3 years. Alternatively, there is, for example, a method in which a drug holiday of about 1 to 14 days is set in the administration period, and the administration is performed through 1 to 36 with 2 days to 3 months considered as one cycle. A method is preferable in which the administration is performed through 3 to 24 cycles with 14 to 30 days considered as one cycle.

The fibroblast growth factor receptor (FGFR) is known to activate the STAT3 channel, the MAPK channel and the PI3K-AKT channel in the downstream due to the genetic abnormalities of FGFR. The activation of these channels promotes growth, survival and migration of cancer cells, which contributes to canceration.

Therefore, the FGFR inhibitor is known to be used for suppressing growth of cancer cells.

FGFR has four types of receptors: FGFR1, FGFR2, FGFR3 and FGFR4. The genetic abnormalities of FGFR1, FGFR2 and FGFR3 have been reported in various tumors.

The fibroblast growth factor (FGF) has 23 types of family members, and activates FGFR signals by binding to FGFR. FGF2 bonds to all FGFRs.

The FGFR inhibitor for use in the present invention means a substance capable of directly neutralizing, blocking, inhibiting, reducing, or preventing the FGFR tyrosine kinase activity. The substance includes a low molecular weight compound, an antibody, an antisense, a transcription inhibitor, a small interfering RNA (siRNA, sgRNA) and the like.

Examples of the non-selective FGFR inhibitor include sorafenib (chemical name: 4-[2-(4-chloro-2-(5-nitro-2-fluorophenyl)phenyl)-1-piperazinyl]-N-(2-pyridyl)benzamide), dopatenib (chemical name: 4-[(4-(4-hydroxy-3-(trifluoromethyl)phenyl)piperazin-1-yl)carbonyl]-2-(3,5-dimethoxyphenyl)benzonitrile), cebrutinib (chemical name: N-(4-4-12-(4-(5-fluoro-2-fluorophenyl)piperazin-1-yl}pyrimidin-4-yl)phenyl)-2-(2-methoxyethoxy)benzamide), darapanib (chemical name: 4-[2-(4-(5-fluoro-2-fluorophenyl)phenyl)-1-piperazinyl]-N-(2-pyridyl)benzamide), votolitinib (chemical name: N-[4-(4-{2-(4-(5-fluoro-2-fluorophenyl)piperazin-1-yl}pyrimidin-4-yl)phenyl]-2-(2-methoxyethoxy)benzamide), and the like. Examples of the FGFR1-3 selective oral tyrosine kinase inhibitor that is clinically used include compounds such as infigratinib (BGJ398, chemical name: N'-(2,6-dichloro-3,5-dimethoxyphenyl)-N-[6-[[4-(4-ethyl-1- piperazinyl)phenyl]amino]-4-pyrimidinyl]-N-methyl-urea), derazantinib (ARQ087, chemical name: (6R)-6-(2-fluorophenyl)-5,6-dihydro-N-[3-[2-[(2-methoxyethyl)amino]ethyl]phenyl]-benzo[h]quinazolin-2-amine), pemigatinib (INCB054828, chemical name: 3-(2,6-difluoro-3,5-dimethoxyphenyl)-1-ethyl-1,3,4,7-tetrahydro-8-(4-morpholinylmethyl)-2H-pyrrolo [3',2':5,6]pyrido[4,3-d]pyrimidin-2-one), erdafitinib (chemical name: N¹-(3,5-dimethoxyphenyl)-N²-(1-methylethyl)-N¹-[3-(1-methyl-1H-pyrazol-4-yl)-6-quinoxalinyl]-1,2-ethanediamine), ponatinib (chemical name: 3-(2-imidazo[1,2-b]pyridazin-3-ylethynyl)-4-methyl-N-[4-[(4-methyl-1-piperazinyl)methyl]-3-(trifluoromethyl)phenyl]-benzamide), Debio1347(chemical name: [5-amino-1-(2-methyl-1H-benzimidazol-6-yl)-1H-pyrazol-4-yl]-1H-indol-2-yl-methanone), futibatinib (TAS-120, chemical name: 1-[(3S)-3-[4-amino-3-[2-(3,5-dimethoxyphenyl)ethynyl]-1H-pyrazolo[3,4-d]pyrimidin-1-yl]-1-pyrrolidinyl]), tasurgratinib, E7090, chemical name: 5-((2-(4-(1-(2-hydroxyethyl)piperidin-4-yl)benzamide)pyridin-4-yl)oxy)-6-(2-methoxyethoxy)-N-methyl-1H-indole-1-carboxyamide), AZD4547(chemical name: N-(5-(3,5- dimethoxyphenethyl)-1H-pyrazol-3-yl)-4-((3S, 5R)-3,5-dimethylpiperazin-1-yl)benzamide), PD173074 (chemical name: 1-(tert-butyl)-3-(2-((4-(diethylamino)butyl)amino)-6-(3,5-dimethoxyphenyl)pyrido[2,3-d]pyrimidin-7-yl)urea).

Infigratinib, pemigatinib, erdafitinib, AZD4547 or a salt thereof is preferable.

These compounds or the salts thereof are manufactured and sold as pharmaceutical preparations or can be obtained as reagents for research, and alternatively, they can be produced by a conventional method. These compounds or the salts thereof also include hydrates, various pharmaceutically acceptable solvates, crystalline polymorphs and the like.

The FGFR inhibitor is formulated according to a conventional method (for example, Remington's Pharmaceutical Science, latest edition, Mack Publishing Company, Easton, U.S.A.) and pharmaceutically acceptable carriers and additives may also be contained. Examples thereof include, but are not limited to, a surfactant, an excipient, a colorant, a flavoring agent, a preservative, a stabilizer, a buffer, a suspending agent, an isotonic agent, a binder, a disintegrant, a lubricant, a flow promoter, and a corrigent, and other commonly used carriers may be used as appropriate. Specifically, suitable examples thereof include trehalose, light anhydrous silicic acid, lactose, crystalline cellulose, mannitol, starch, carmellose calcium, carmellose sodium, hydroxypropyl cellulose, hydroxypropyl methylcellulose, polyvinyl acetal diethylamino acetate, polyvinylpyrrolidone, gelatin, a medium chain triglyceride, polyoxyethylene hydrogenated castor oil 60, refined sugar, carboxymethyl cellulose, corn starch, an inorganic salt, and a polysorbate.

In the present invention, the method for administering the FGFR inhibitor may be carried out by either oral or parenteral administration. Particularly preferred is an administration method by oral administration, and specifically, suitable examples of such administration method include administration by a liquid, a powder, granules, a tablet, an enteric coated drug, a capsule or the like. As an example of administration by injection, the therapeutic drug of the present invention may be administered systemically or locally by intravenous injection, intramuscular injection, intraperitoneal injection, subcutaneous injection, or the like. In addition, the method of administration may be appropriately selected according to the patient's age and symptoms. For example, in the case of an oral agent, the dose may be selected within the range of 0.1 mg/kg to 100 mg/kg, preferably 0.1 mg/kg to 5 mg/kg per administration. For example, the dose may be selected within the range of 9 mg to 150 mg per patient. However, the dose of the FGFR inhibitor used in the present invention is not limited to these doses.

Depending on the type and severity of the disease, for example, the preferred daily dose of infigratinib is within the range of 75 to 125 mg/kg, but is not limited thereto, and may be gradually reduced depending on the degree of symptoms and side effects. For the frequency of administration, an administration once a day for a predetermined period of time (for example, 3 weeks) is usually considered as one cycle, followed by a predetermined drug holiday (for example, 1 week), and then the cycle is repeated. The number of cycles varies depending on the type and severity of the disease. The treatment is maintained until target cancer is treated or the desired therapeutic effect is achieved by measuring according to a method known in the art. In one example, infigratinib is administered at a dose of 125 mg once daily for 3 consecutive weeks, and in case side effects or the like are observed, the dose is reduced to 100 mg as a primary dose reduction and to 75 mg as a secondary dose reduction. However, other dosage regimens may also be useful.

The FGFR inhibitor can be administered until the target cancer is treated or the desired therapeutic effect is achieved, with the above 3-week administration and 1-week drug holiday considered as one cycle. Specifically, it can be administered over 1 to 36 cycles.

For example, erdafitinib is administered at a starting dose of 8 mg once daily, and the dose is increased to 9 mg while the serum phosphate level is monitored.

### Drug, Combination, Pharmaceutical Composition, and Method of Treatment and Prevention

An aspect of the present invention is a drug for treating or preventing ALK fusion gene-positive cancer positive for FGFR1 expression and positive for FGF2 expression at baseline (hereinafter, referred to as target cancer), the drug comprising a compound having ALK inhibitory activity and a FGFR inhibitor in combination.

In the above aspect, "a drug for treating or preventing target cancer, comprising a compound having ALK inhibitory activity and a FGFR inhibitor in combination" means a drug comprising a compound having ALK inhibitory activity and a FGFR inhibitor in combination for simultaneous, separate, or sequential administration in the treatment or prevention of target cancer. The drug of the present invention can also be provided in the form of a compounded drug comprising both a compound having ALK inhibitory activity and a FGFR inhibitor. In addition, the preparation comprising the compound having ALK inhibitory activity and the preparation comprising the FGFR inhibitor may be provided separately, and these preparations may be used simultaneously or sequentially.

In the present application, the term "in combination" specifically includes the statement of use in combination with other drugs in the dosage and administration of a pharmaceutical preparation. Specifically, the statement of use in combination with a FGFR inhibitor in a package insert for a pharmaceutical preparation of a compound having ALK inhibitory activity, or the statement of use in combination with a compound having ALK inhibitory activity in a package insert for a pharmaceutical preparation of a FGFR inhibitor is included.

In the present invention, simultaneous administration refers to the use of a compound having ALK inhibitory activity and a FGFR inhibitor by administering them at the same time, and it may be administered as a compounded drug, administered as a mixture prepared at the time of administration, or preparations of another form may be administered at the same time. When used by simultaneous administration, it may be administered by different routes or it may be administered by the same route, and the dosage forms for administration may be the same or different.

In the present invention, when the compound having ALK inhibitory activity and the FGFR inhibitor are administered separately, the order of administration of the compound having ALK inhibitory activity and the FGFR inhibitor may be: the compound having ALK inhibitory activity is administered after the administration of the FGFR inhibitor; the compound having ALK inhibitory activity and the FGFR inhibitor are administered simultaneously; or the FGFR inhibitor is administered after the administration of the compound having ALK inhibitory activity.

Preferably, the compound having ALK inhibitory activity and the FGFR inhibitor are administered simultaneously.

Specifically, use in combination with a FGFR inhibitor is stated in a package insert for a pharmaceutical preparation of a compound having ALK inhibitory activity, and the administration is performed in accordance with the dosage and administration of the compound having ALK inhibitory activity as specified in the case of use in combination.

Specifically, use in combination with a compound having ALK inhibitory activity is stated in a package insert for a pharmaceutical preparation of a FGFR inhibitor, and the administration is performed in accordance with the dosage and administration of the compound having ALK inhibitory activity as specified in the case of use in combination.

In the above drug, when the compound having ALK inhibitory activity and the FGFR inhibitor are contained and provided in separate preparations, the dosage form of these preparations may be the same or different. For example, both may have a different dosage form than the other, selected from an oral agent, a parenteral agent, an injection, a drip infusion, and an intravenous drip infusion, or both may have the same dosage form selected from an oral agent, a parenteral agent, an injection, a drip infusion, and an intravenous drip infusion. Preferably, the dosage form of both is an oral agent. In addition, the above drug may further be combined with one or more different preparations.

Note that, in the present invention, the pharmaceutical composition comprises the compound having ALK inhibitory activity and/or the FGFR inhibitor used in the treatment and/or prevention of the present invention, and may further comprise a pharmaceutically acceptable carrier.

In the present invention, the term "pharmaceutically acceptable carrier" means one or more compatible solid or liquid diluent excipients or encapsulating materials, as previously exemplified, that are suitable for administration to mammals.

In the present invention, a "combination agent" means a form in which a compound having ALK inhibitory activity and a compound that is a FGFR inhibitor can be used separately for the same subject, or a combination of these compounds where each is formulated without mixing them. For example, packaged doses of the drug are included so that a pharmaceutical preparation of a compound having ALK inhibitory activity and a pharmaceutical preparation of a FGFR inhibitor can be taken simultaneously.

In the present invention, the "pharmaceutical preparation" comprising a compound having ALK inhibitory activity and a FGFR inhibitor in combination includes solid preparations such as tablets, capsules, granules, powders, and pills; liquids such as aqueous and non-aqueous oral solutions and suspensions, and parenteral solutions filled in a container adapted for subdividing into individual doses; lyophilized preparations which can be used by dissolving at the time of use; or preparations combining any of these dosage forms, in which these active ingredients are formulated separately, or comprised in the same preparation. The pharmaceutical preparation comprises, per unit dosage form, 150 mg to 800 mg, preferably 150 mg to 400 mg, and particularly preferably 150 mg to 300 mg of alectinib or a salt thereof in free form. Separate preparations of alectinib or a salt thereof include, specifically, a 150 mg capsule preparation, 150 mg, 300 mg, 600 mg tablets and the like.

In another viewpoint, the present invention provides a drug for treating or preventing target cancer, the drug comprising a compound having ALK inhibitory activity as an active ingredient, which is used in combination with a FGFR inhibitor. "A drug for treating or preventing target cancer, the drug comprising a compound having ALK inhibitory activity as an active ingredient, which is used in combination with a FGFR inhibitor" means a drug for use in treating or preventing target cancer, the drug comprising a compound having ALK inhibitory activity as an active ingredient, provided that it is used in combination with a FGFR inhibitor. When the drug comprising a compound having ALK inhibitory activity as an active ingredient of the present invention is used in combination with a FGFR inhibitor, the drug may be administered simultaneously with the FGFR inhibitor, or may be administered before or after the administration of the FGFR inhibitor. If a compound having ALK inhibitory activity is administered before or after the administration of a FGFR inhibitor, the timing of the administration may be optimized by measuring the residual concentration of the FGFR inhibitor in the subject. The concentration can be determined based on the results of analyzing a sample collected from the subject by an immunoassay such as ELISA described below, which is publicly known to those skilled in the art.

In another viewpoint, the present invention provides a drug for treating or preventing target cancer, the drug comprising a FGFR inhibitor as an active ingredient, which is used in combination with a compound having ALK inhibitory activity. "A drug for treating or preventing target cancer, the drug comprising a FGFR inhibitor as an active ingredient, which is used in combination with a compound having ALK inhibitory activity" in the present invention means a drug for use in treating or preventing target cancer, the drug comprising a FGFR inhibitor as an active ingredient, provided that it is used in combination with a compound having ALK inhibitory activity. When the drug comprising a FGFR inhibitor as an active ingredient is used in combination with a compound having ALK inhibitory activity, the drug may be administered simultaneously with the compound having ALK inhibitory activity, or may be administered before or after the administration of the compound having ALK inhibitory activity. If a FGFR inhibitor is administered before or after the administration of a compound having ALK inhibitory activity, the timing of the administration may be optimized by measuring the residual concentration of the compound having ALK inhibitory activity in the subject. The concentration can be determined based on the results of separating a sample collected from the subject using a separation device such as various types of chromatography, then analyzing using a method of analysis publicly known to those skilled in the art.

The above invention means that a compound having ALK inhibitory activity and a FGFR inhibitor are administered or used (hereinafter, simply referred to as "administered") together, and the order of administration, interval of administration, and the like shall not be construed as limited. The invention may also be used as a product in which a compound having ALK inhibitory activity is combined with a FGFR inhibitor. Furthermore, when a compound having ALK inhibitory activity is used in combination with a FGFR inhibitor in the present invention, each may be administered at a dose less than that at which either one is used alone, if desired.

### Cancer Type

The drug of the present invention is useful for the prevention or treatment of diseases such as various cancers such as leukemia (acute myelocytic leukemia, chronic myelocytic leukemia, acute lymphocytic leukemia, chronic lymphocytic leukemia and the like), malignant lymphoma (Hodgkin's lymphoma, non-Hodgkin's lymphoma and the like), brain tumor, neuroblastoma, glioma, thyroid cancer, myelodysplastic syndrome, head and neck cancer, esophageal cancer, stomach cancer, bowel cancer, colorectal cancer, breast cancer, ovarian cancer, lung cancer, pancreatic cancer, liver cancer, gallbladder cancer, skin cancer, malignant melanoma, kidney cancer, pyeloureteral cancer, bladder cancer, uterine cancer, testicular cancer, and prostate cancer. Furthermore, the compound of the present invention is useful for the prevention or treatment of the invasion and metastasis of solid cancer.

In particular, the drug of the present invention is useful for the prevention or treatment of ALK fusion gene-positive cancer. Examples of the ALK fusion gene-positive cancer include, but are not limited to, non-Hodgkin's lymphoma, neuroblastoma, inflammatory myofibroblastic cytoma, kidney cancer and lung cancer.

In the present invention, being "ALK-fusion gene-positive" means that a fusion gene of ALK and another gene is present. Examples of the other fusion gene include, but are not limited to, echinoderm microtubule-associated protein like protein 4 (EML 4, GeneBank ^{™} accession number NM_019063), TFG (TRK-fused gene, GeneBank ^{™} accession number accession number NM_006070).

The method for identifying ALK fusion gene-positive cancer is described in WO2008/127248, Japanese Patent Laid-Open No. 2008-295444, "Guidance for ALK Gene Testing" (Biomarker Committee, the Japan Lung Cancer Society), and the like. As Kits for detecting such a fusion gene, Vysis LSI ALK Break Apart Rearrangement Probe Kit ^{™} (Abbott) and Histofine ALK iAEP TM Kit (Nichirei Corporation) are commercially available.

"ALK sensitivity" means having sensitivity to ALK inhibitor. Specifically, it means that when an ALK inhibitor is once applied to a patient with ALK fusion gene-positive cancer or ALK fusion gene-positive cancer cells, a certain level of reducing action or growth suppressive action is exhibited as compared to a control such as a solvent or as compared to a state before administration of the ALK inhibitor.

Being "positive for FGFR1 expression" or "positive for FGF2 expression" at baseline means that the level of messenger RNA (mRNA) encoding FGFR1 protein or FGF2 protein is higher as compared to normal cells. The normal cells for comparison are those identical in histological type, in particular, phenotype to a tumor, or those from a phenotype in which the tumor is generated.

A known method for determining a mRNA level or a level of protein can be used to determine that the cells are "positive for FGFR1 expression" or "positive for FGF2 expression" at baseline.

Methods suitable for determining a gene expression level by a mRNA level include, but are not limited to, standard assays for determining an expression level of mRNA, such as qPCR, RT-PCR, RNA protection analysis, Northern blotting, RNA dot blotting, in situ hybridization, microarray techniques, methods using a tag (serial analysis of gene expression (SAGE) including variations such as LongSAGE and SuperSAGE, and the like), microarray, and fluorescence in situ hybridization (FISH) (including variations such as Flow-FISH, qFiSH, and double fusion

### FISH (D-FISH)).

Specifically, the determination can be made by, for example, the following method.

The FGFR1 and FGF2 mRNA expression levels in a tumor tissue sample are quantified by RNA in situ hybridization using a FGFR1 or FGF2 probe. The in situ hybridization is known in the art, as described in, for example, Wang et al., J Mol Diagn. 2012 Jan; 14(1): 22-9.

The in situ hybridization is performed typically on cells or a tissue section fixed on a slide, and a direct method and an indirect method are used.

In the direct method, a detectable molecule (for example, a fluorophore, i.e., fluorescence in situ hybridization or FISH) is directly bound to a nucleic acid probe so that a probe-target hybrid can be visualized under a microscope immediately after the hybridization reaction. In this method, it is extremely important that the probe-reporter bond withstands very severe hybridization conditions and washing conditions. It is important that the reporter molecule does not interfere with the hybridization reaction. Examples of the method satisfying these criteria include a method of fluorochrome-labeling the end of a RNA probe, which was developed by Bauman et al. (1980, 1984), and a method of directly enzyme-labeling a nucleic acid, which was described by Renz and Kurz (1984), and a radioactive label may also be used. In the indirect method, it is necessary that the probe contain a detectable molecule introduced chemically or enzymatically, and the detection can be performed by, for example, a method using a biotin-streptavidin system. An ISH probe for detecting the expression of FGFR1 or FGF2 mRNA is designed in accordance with, for example, Jin and Lloyd (J Clin Lab Anal. 1997; 11(1): 2-9). The sequence used for designing a probe according to the present invention is a sequence having Gen Bank sequence accession number NM_023110.2 (FGFR1). A poly (A) tail provided under the above-described Gen Bank sequence accession number is not used for designing a probe. As a method for formalin fixation, a paraffin-embedded tissue sample or a frozen sample, a known method can be used. For bright field microscopy, conventional fluorescent dyes for color forming dyes or multiplex analysis may be used (Levsky and Singer, J Cell Sci.2003 Jul 15; 116(Pt 14): 2833-8).

Preferably, the FGFR1 and FGF2 mRNA expression levels in a tumor tissue sample can be measured by RNAscope (registered trademark) (Advanced Cell Diagnostics, Inc., Newark, CA, USA) using RNA in situ hybridization using a technique from ACD (Advanced Cell Diagnostics, Inc., 3960 Point Eden Way, Hayward, CA 94545, USA). RNAscope is a commercially available fully automated multiple assay, which is a sensitive and specific assay in which maximum four types of different RNAs per slide are detected, and one RNA in a fresh-frozen or fix-frozen FFPE tissue specimen is detected (Journal pathology, 2021. 254(4): p 405-417).

The determination of being "positive for FGFR1 expression" or "positive for FGF2 expression" at baseline includes, but is not limited to, a conventional method for determining an expression level of protein, for example, using an antibody capable of binding specifically to a protein encoded by the gene (or a fragment thereof containing an antigen determinant group), followed by quantification of the resulting antibody-antigen complex. For example, the protein level of FGFR1 or FGF2 can be quantified by using a standard assay for determining an expression level of protein, such as Western blotting, Western transfer, ELISA (enzyme linked immunosorbent assay), RIA (radioimmunoassay), competitive EIA (competitive enzyme immunoassay), DAS-ELISA (double antibody sandwich ELISA), an immunocytochemical technique, an immunohistochemical technique, a technique based on a biochip of protein containing a specific antibody or a microarray, or an assay based on colloid precipitations in the form or dip sticks or the like.

The antibodies used in these assays may be any of, for example, polyclonal serum, a hybridoma supernatant, a monoclonal antibody, an antibody fragment. Fv, Fab, Fab', F(ab')2, ScFv, a diabody, a triabody, a tetrabody and a humanized antibody. At the same time, the antibody may, but is not required to, be labeled. Exemplary but non-exclusive examples of the marker that can be used include radioactive isotopes, enzymes, fluorophores, chemiluminescent reagents, enzyme substrates or coenzymes, enzyme inhibitors, particles, colorants, and the like. There are a wide range of various well known assays that can be used in the present invention, where a non-labeled antibody (primary antibody) and a labeled antibody (secondary antibody) are used. These techniques include Western blotting, Western transfer, ELISA, RIA, competitive EIA, DAS-ELISA, an immunocytochemical technique, an immunohistochemical technique, a technique based on a biochip of protein containing a specific antibody or a microarray, or an assay based on colloid precipitations in the form of dip sticks or the like. Other methods for detecting and quantifying a level of target protein include affinity chromatography, a binding ligand assay, and the like.

Specifically, when the Western blotting method is used, FGFR1 or FGF2 protein separated by electrophoresis is transferred to a membrane, and the presence of the protein can be detected using an antibody to the protein.

When the Western blotting method is used, being "positive for FGFR1 expression" at baseline means cells in which expression of FGFR1 protein is more intensely detected as compared to a control (SNU-2535 cells) in Western blotting. Being "positive for FGF2 expression" at baseline means cells in which expression of FGF2 protein is more intensely detected as compared to a control (SNU-2535 cells) in Western blotting.

The quantification of the FGFR1 or FGFR2 protein level by a immunostaining method can be performed constructing a tissue microarray (TMA) containing an assembled sample from a subject, and determining an expression level of the relevant protein by an immunohistochemical technique. The immunostaining intensity may be evaluated by two or more persons for maintaining the reproducibility of the method, and scored using consistent and clear cutoff criteria. Inconsistency can be solved by performing reevaluation at the same time. For example, the results of immunostaining can be recorded as being negative for expression (0) versus being positive for expression, and low expression (1+) versus middle expression (2+) and high expression (3+) on the basis of specific cutoffs. As general criteria, cutoffs are selected for promoting reproducibility, and translating biological events if possible. Alternatively, the immunostaining intensity can be evaluated by using an imaging technique or an automated method (that disclosed in Rojo, M.G. et al. (Folia Histochem. Cytobiol. 2009; 47: 349-54), Mulrane, L. et al.(Expert Rev. Mol. Diagn. 2008; 8: 707-25), or the like).

In another aspect of the present invention, there is provided a method of enhancing the therapeutic effect of a compound having ALK inhibitory activity in the treatment of a cancer patient by the compound having ALK inhibitory activity, by using a FGFR inhibitor. Here, enhancing the therapeutic effect means either that the treatment success rate increases as compared to the case of single-agent administration; the amount of compound having ALK inhibitory activity administered for treatment is reduced as compared to the case of single administration; a therapeutic effect is shown in more severe cases, for example, in patients with prior treatment failure or exacerbation; the duration of treatment with the compound having ALK inhibitor activity is shorter than the expected duration of treatment with the compound alone or the duration of treatment planned before starting the treatment by combined administration, due to the disappearance of the cancer or the like; or that the duration of treatment including the compound having ALK inhibitory activity and/or other maintenance therapy is prolonged by the suppression of disease progression. In addition, in another aspect of the present invention, there is provided a method of prolonging the progression-free survival (PFS) in a subject, comprising administering an effective amount of a compound having ALK inhibitory activity and an effective amount of a FGFR inhibitor in combination. In addition, in another aspect of the present invention, there is provided a method of using a compound having ALK inhibitory activity or a FGFR inhibitor for producing a pharmaceutical composition for treating or preventing target cancer, comprising the compound having ALK kinase inhibitory activity and the FGFR inhibitor as active ingredients.

On the basis of the findings of the present invention, it has also been confirmed that similarly to the compound having ALK inhibitory activity, the effect of an EGFR inhibitory, a HER2 inhibitor or a BRAF inhibitor on ALK fusion gene-positive cancer, in particular, ALK gene-positive cancer positive for FGFR1 expression and positive for FGF2 expression at baseline, can be enhanced by using the inhibitor in combination with a FGFR inhibitor. Examples of the EGFR inhibitor include epirubicinitib, gefitinib, afatinib, erlotinib, dasatinib, osimertinib, and the like. Examples of the HER2 inhibitor include neratinib, trastuzumab, lapatinib, pertuzumab, adavopatib, acetretinib, panitumumab, and the like. Examples of the BRAF inhibitor include dabrafenib, trametinib, vemurafenib, cobimetinib, encorafenib, ubekinumab, and the like.

Examples of the preferred combination of the EGFR inhibitor and the FGFR inhibitor include epirubicinitib/infigratinib, epirubicinitib/pemigatinib, epirubicinitib/erdafitinib, epirubicinitib/AZD4547, epirubicinitib/tasurgratinib, gefitinib/infigratinib, gefitinib/pemigatinib, gefitinib/erdafitinib, gefitinib/AZD4547, gefitinib/tasurgratinib, afatinib/infigratinib, afatinib/pemigatinib, afatinib/erdafitinib, afatinib/AZD4547, afatinib/tasurgratinib, erlotinib/infigratinib, erlotinib/pemigatinib, erlotinib/erdafitinib, erlotinib/AZD4547, erlotinib/tasurgratinib, dasatinib/infigratinib, dasatinib/pemigatinib, dasatinib/erdafitinib, dasatinib/AZD4547, dasatinib/tasurgratinib, osimertinib/infigratinib, osimertinib/pemigatinib, osimertinib/erdafitinib, osimertinib/AZD4547, and osimertinib/tasurgratinib. Erlotinib/infigratinib, erlotinib/AZD4547, osimertinib/infigratinib, and osimertinib/AZD4547 are particularly preferable.

Examples of the preferred combination of the HER2 inhibitor and the FGFR inhibitor include neratinib/infigratinib, neratinib/pemigatinib, neratinib/erdafitinib, neratinib/AZD4547, neratinib/tasurgratinib, trastuzumab/infigratinib, trastuzumab/pemigatinib, trastuzumab/erdafitinib, trastuzumab/AZD4547, trastuzumab/tasurgratinib, lapatinib/infigratinib, lapatinib/pemigatinib, lapatinib/erdafitinib, lapatinib/AZD4547, lapatinib/tasurgratinib, pertuzumab/infigratinib, pertuzumab/pemigatinib, pertuzumab/erdafitinib, pertuzumab/AZD4547, pertuzumab/tasurgratinib, adavopatib/infigratinib, adavopatib/pemigatinib, adavopatib/erdafitinib, adavopatib/AZD4547, adavopatib/tasurgratinib, acetretinib/infigratinib, acetretinib/pemigatinib, acetretinib/erdafitinib, acetretinib/AZD4547, acetretinib/tasurgratinib, panitumumab/infigratinib, panitumumab/pemigatinib, panitumumab/erdafitinib, panitumumab/AZD4547, and panitumumab/tasurgratinib. Neratinib/infigratinib, neratinib/AZD4547, lapatinib/infigratinib, and lapatinib/AZD4547 are particularly preferable.

Examples of the preferred combination of the BRAF inhibitor and the FGFR inhibitor include dabrafenib/infigratinib, dabrafenib/pemigatinib, dabrafenib/erdafitinib, dabrafenib/AZD4547, dabrafenib/tasurgratinib, trametinib/infigratinib, trametinib/pemigatinib, trametinib/erdafitinib, trametinib/AZD4547, trametinib/tasurgratinib, vemurafenib/infigratinib, vemurafenib/pemigatinib, vemurafenib/erdafitinib, vemurafenib/AZD4547, vemurafenib/tasurgratinib, cobimetinib/infigratinib, cobimetinib/pemigatinib, cobimetinib/erdafitinib, cobimetinib/AZD4547, cobimetinib/tasurgratinib, encorafenib/infigratinib, encorafenib/pemigatinib, encorafenib/erdafitinib, encorafenib/AZD4547, encorafenib/tasurgratinib, ubekinumab/infigratinib, ubekinumab/pemigatinib, ubekinumab/erdafitinib, ubekinumab/AZD4547, and ubekinumab/tasurgratinib. Dabrafenib/infigratinib, dabrafenib/AZD4547, trametinib/infigratinib, and trametinib/AZD4547 are particularly preferable.

In the present invention, comprising a compound having ALK inhibitory activity and/or a FGFR inhibitor as an active ingredient means comprising a compound having ALK inhibitory activity and/or a FGFR inhibitor as a main active ingredient, and does not limit the content of the compound having ALK inhibitory activity and/or the FGFR inhibitor. In addition, the term "treatment" means that the administration of the drug according to the present invention to a subject causes either the death of cancer cells or a decrease in the number of those cells, the suppression of the growth of cancer cells, the suppression of the metastasis of cancer cells, the extension of progression-free survival (PFS) as compared to the case of monotherapy, or the improvement of various symptoms caused by target cancer. In addition, the word "prevention" means either preventing an increase in the number of cancer cells that had been reduced from when they grow again, or preventing the regrowth of cancer cells whose growth had been suppressed.

In the present invention, the "effective amount" means the daily dose of each inhibitor when a compound having ALK inhibitory activity and a FGFR inhibitor are administered in combination. The dose in the present invention may be the same as the dose when each inhibitor is used alone, or it may be a lower dose than the dose when each inhibitor is used alone. Alternatively, the effective amount in the present invention is the same as the dose when one is used alone, and it may be a lower dose than the dose when the other is used alone.

Another aspect of the present invention includes a drug for suppressing the acquisition of resistance by ALK fusion gene-positive cancer positive for FGFR1 expression and positive for FGF2 expression at baseline, the drug comprising an ALK inhibitor and a FGFR inhibitor in combination; a drug for suppressing the acquisition of resistance by ALK fusion gene-positive cancer positive for FGFR1 expression and positive for FGF2 expression at baseline, the drug comprising an ALK inhibitor as an active ingredient, which is used in combination with a FGFR inhibitor; a drug for suppressing the acquisition of resistance by ALK fusion gene-positive cancer positive for FGFR1 expression and positive for FGF2 expression at baseline, the drug comprising a FGFR inhibitor as an active ingredient, which is used in combination with an ALK inhibitor; and a method for suppressing the acquisition of resistance by ALK fusion gene-positive cancer positive for FGFR1 expression and positive for FGF2 expression at baseline, the method comprising administering to a subject an effective amount of compound having ALK inhibitory activity and an effective amount of a FGFR inhibitor in combination.

A drug or method for suppressing the acquisition of resistance by ALK fusion gene-positive cancer positive for FGFR1 expression and positive for FGF2 expression at baseline refers to a drug or method capable of suppressing the acquisition of resistance by using an ALK inhibitor and a FGFR inhibitor in combination on ALK fusion gene-positive cancer positive for FGFR1 expression and positive for FGF2 expression at baseline which has not received prior treatment with an ALK inhibitor or the like. "Suppressing the acquisition of resistance" means that the sensitivity to the ALK inhibitor or the cell growth suppressive action in an *in vivo* test using cultures of tumor cells acquired from a subject at arbitrary time points before and after the start of treatment, where the subject is treated with a combination of the ALK inhibitor and the FGFR inhibitor (for example, Anticancer Drugs. 2022 Feb 1; 33(2): 124-131) is compared, and the sensitivity of the tumor cells or the cell growth suppressive action at the arbitrary time point after the start of treatment is equivalent or does not significantly decrease as compared to that at the time point of the start of treatment.

In the present invention, "progression-free survival (PFS)" refers to the time from treatment (or randomization) to first disease progression or death. In one aspect of the present invention, PFS can be assessed by the Response Evaluation Criteria in Solid Tumors (RECIST). In one aspect of the present invention, PFS can be assessed by CA-125 levels as a determinant of progression.

In the present invention, specific examples of "prolonging progression-free survival" include the use of a compound having ALK inhibitory activity in combination with a FGFR inhibitor to prolong PFS compared to the PFS when the compound having ALK inhibitory activity or the FGFR inhibitor is administered as a single agent at the same dose used when in combination.

In the present invention, the "subject" means, but is not limited to, a mammal including a human or a non-human mammal, for example, a cow, a horse, a dog, a sheep, or a cat, that is the subject of administration of the drug of the present invention or requires the administration of the drug of the present invention. Preferably, the subject is human. The subject includes a patient (including human and non-human mammal). Specifically, these are patients having, or humans or non-human mammals likely to have, various cancers such as leukemia (acute myelocytic leukemia, chronic myelocytic leukemia, acute lymphocytic leukemia, chronic lymphocytic leukemia and the like), malignant lymphoma (Hodgkin's lymphoma, non-Hodgkin's lymphoma and the like), brain tumor, neuroblastoma, glioma, thyroid cancer, myelodysplastic syndrome, head and neck cancer, esophageal cancer, stomach cancer, bowel cancer, colorectal cancer, breast cancer, ovarian cancer, lung cancer, pancreatic cancer, liver cancer, gallbladder cancer, skin cancer, malignant melanoma, renal cancer, pyeloureteral cancer, bladder cancer, uterine cancer, testicular cancer, prostate cancer, and the tumors metastasized from these tumors.

### Product

As another aspect of the present invention, a product is provided. In one embodiment, a product is provided for treating or preventing target cancer in a subject, and comprises: (a) (1) a preparation comprising a FGFR inhibitor, (2) a container, and (3) an instruction or label indicating that the FGFR inhibitor and at least one compound having ALK inhibitory activity are administered in combination to a subject for treating target cancer in the subject; or (b) (1) a preparation comprising a compound having ALK inhibitory activity, (2) a container, and (3) an instruction or label indicating that the compound having ALK inhibitory activity and at least one FGFR inhibitor are administered in combination to a subject for treating target cancer in the subject.

The product comprises a container comprising a preparation comprising a FGFR inhibitor or a compound having ALK inhibitory activity.

The product may further comprise a label or instruction on or accompanying the container.
In the present invention, the "instruction" means a document normally included in the commercial packaging of a preparation, which includes information on the indications, usage, dose, administration, contraindications and/or warnings regarding the use of the preparation. The "label" means a sheet-shaped article containing the indication of the product name, dosage, dosage form, indications, and the like of a preparation comprising a FGFR inhibitor or a compound having ALK inhibitory activity, which is affixed directly to the container.

The label or instruction indicates the indications of the preparation, i.e., that it is to be used for the treatment of target cancer and the like. In one embodiment, the label or instruction indicates that the preparation can be used to treat target cancer, and the like. The label or instruction may also indicate that the preparation can be used to treat other disorders.

Examples of suitable containers include PTP, a bottle, a vial, a syringe, and a blister pack. The container may be formed from a variety of materials such as glass or plastic. These containers may be further packaged in a paper outer box on which the content of the above label and the like are printed.

Still another aspect of the present invention is a method for selecting a patient with ALK fusion gene-positive cancer positive for FGFR1 expression and positive for FGF2 expression at baseline.

The method comprises acquiring a tumor tissue of a patient diagnosed as ALK fusion gene-positive cancer, and measuring expression levels of FGFR1 and FGF2 in the tumor tissue specimen. As the expression levels of FGFR1 and FGF2, expression levels of protein or expression levels of mRNA can be used. Specifically, the above-described method for determination of being "positive for FGFR1 expression" or "positive for FGF2 expression" at baseline can be used.

The tumor tissue acquired from a patient diagnosed as ALK fusion gene-positive cancer may be used as a fresh-frozen, fix-frozen or formalin-fixed paraffin-embedded (FFPE) specimen in the method.

### Examples

All patents and references expressly cited herein are incorporated herein by reference in their entirety.

Hereinafter, the present invention will be specifically described with reference to the descriptions of Examples, but the present invention is not to be construed as limited by these descriptions.

The drugs, reagents and cells used in Examples are as follows.

Alectinib was synthesized by Chugai Pharmaceutical Co., Ltd. (Tokyo, Japan). Infigratinib (BGJ398) was obtained from LC Laboratories (Woburn, MA, USA). AZD4547 was obtained from ChemScene (Monmouth Junction, NJ, USA). Lorlatinib was obtained from Selleck Chemicals LLC (Houston, TX, USA). PPP was obtained from Santa Cruz Biotechnology, Inc. (Dallas, TX, USA). All the drugs were dissolved in dimethyl sulfoxide (DMSO: Sigma-Aldrich, St. Louis, MO, USA) for *in vitro* assays, and in a 6% (w/v) solution of Captisol (ChemScene) for *in vivo* assays. In the assays, DMSO and Captisol were used as solvent controls. Recombinant human FGF1, FGF2, FGF7 and FGF12 proteins were obtained from R&D Systems (Minneapolis Company, MN, USA), and dissolved in phosphate buffer physiological saline (PBS, Sigma-Aldrich Co. LLC). Recombinant human FGF9 and FGF18 protein molecules were obtained from Abcam (Cambridge, England), and dissolved in distilled water. Recombinant human FGF11 protein molecules were obtained from LifeSpan Biosciences, Inc. (Seattle, WA, USA), and dissolved in a Tris buffer solution (Horizon Discovery in Cambridge, England).

Tissues, suppliers and culture media for human cancer cell lines used in Examples are as follows. The cells were cell-cultured at 37°C under 5% CO₂.

**Table 1**

| Cell line | Tissue | Oncogenic driver mutation | Supplier | Culture medium |
|---|---|---|---|---|
| NCl-H2228 | NSCLC | EML4-ALK¹ | ATCC | 10%FBS-RPMI1640 |
| SNU-2535 | NSCLC | EML4-ALK, G1269A² | KCLB | 10%FBS-RPMI1640 |

The *in vitro* data was statistically analyzed using the Student's t-test or the Dunnett test. The *in vivo* data was statistically analyzed using the Wilcoxon rank sum test with the Holm-Bonferroni method. The threshold of significance was set to P < 0.05.

The [numbers] in Examples correspond to the numbers of references described later.

### Example 1

NCI-H2228 cells (parental cells) were treated with alectinib for 13 days to prepare DTP cells having transient resistance to alectinib. The drug tolerant persister (DTP) cell means a cell that has acquired drug resistance without genetic mutation through a certain period of drug exposure. The cell has reversibility such that it comes to exhibit drug sensitivity again as drug exposure is stopped.

First, a screening test for the growth suppressive action on parental cells and DTP cells was conducted using an anticancer agent library of 3114 compounds including various compounds that inhibit signals (TargetMol). The results showed that nine compounds strongly suppressed the growth of DTP cells while having no influence on the growth of parental cells (Table 2). Next, for these nine compounds, a cell growth suppression test was conducted, and the ratio of IC₅₀ of DTP cells with respect to the parental line was calculated. The results showed that among the nine compounds, erdafitinib as a FGFR1 inhibitor exhibited the most potent cell growth suppressive effect on DTP cells, and therefore it was indicated that in DTP cells, FGFR1 signals were most strongly activated, and contributed to cell growth (Table 3).

**Table 2**

| No. | Compounds | Anti-proliferative effect on DTP cells | Targets |
|---|---|---|---|
| 1 | THZ531 | 0.47 | CDK12,CDK13 |
| 2 | Hydrocortisone | 0.62 | Glucocorticoid Receptor |
| 3 | Afatinib | 0.65 | EGFR (L858R); EGFR (L858R/T790M); EGFR (wt); HER2 |
| 4 | Poziotinib | 0.67 | HER1; HER2; HER4 |
| 5 | APY29 | 0.68 | IRE1α |
| 6 | Dexamethasone | 0.68 | Annexin A1; Glucocorticoid Receptor; IL receptor; iNOS |
| 7 | Triamcinolone | 0.69 | Glucocorticoid Receptor |
| 8 | AZD6482 | 0.69 | DNA-PK; PI3Kα; PI3Kβ; PI3Kγ; PI3Kδ |
| 9 | Erdafitinib | 0.69 | FGFR1; FGFR2; FGFR3; FGFR4 |

**Table 3**

| Compounds | IC₅₀ | | Ratio of parental to DTP cells |
|---|---|---|---|
| | DTP cells | Parental cells | |
| Erdafitinib | 6.7 | 6136.4 | 920.8 |
| APY29 | 444.8 | 1700.2 | 3.8 |
| Poziotinib | 1388.7 | 3915.8 | 2.8 |
| AZD6482 | 4571.3 | 9873.5 | 2.2 |
| THZ531 | 154.4 | 270.5 | 1.8 |
| Afatinib | 5022.5 | 6433.5 | 1.3 |
| Dexamethasone | 1322.1 | >10000 | ND |
| Triamcinolone | 1364.3 | >10000 | ND |
| Hydrocortisone | 2156.2 | >10000 | ND |

| | | | |
|---|---|---|---|
| ND: not determined because the IC₅₀ for parent cells cannot be calculated. | | | |

Next, a FGFR inhibitor that inhibits FGFR1, FGFR2 and FGFR3, and inhibits FGFR4 to a degree 38 times lower as compared to other FGFRs (BGJ398) [3], or an IGF1R inhibitor (picropodophyllin, PPP) [4], was used to evaluate the growth suppressive effect on DTP cells in accordance with a known method (Anticancer Drugs. 2022 Feb 1; 33(2): 124-131.).

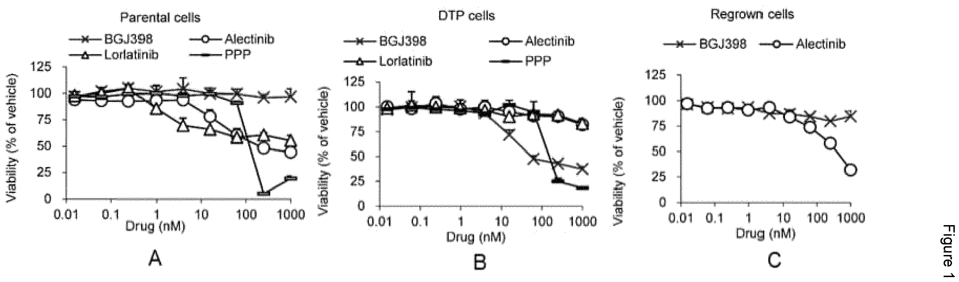

The results are shown below (Figure 1 and Table 4).

**Table 4**

| A | Cells | Alectinib (nM) | Lorlatinib (nM) | BGJ398 (nM) | PPP (nM) |
|---|---|---|---|---|---|
| | Parental cells | 219.6 | 3.8 | >1000 | 151.7 |
| | DTP cells | >1000 | >1000 | 57.8 | 182.5 |
| | Regrown cells | 480.0 | ND | >1000 | ND |

The results showed that NCI-H2228 cells exhibited sensitivity to the ALK inhibitor (alectinib and lorlatinib), and did not exhibit sensitivity to the FGFR inhibitor (BGJ398), whereas DTP cells did not exhibit sensitivity to the ALK inhibitor, and exhibited sensitivity to the FGFR inhibitor.

### Western blotting method

The cells were seeded on a 6-well plate, and on the following day, a drug was added, followed by culturing for a certain time. A Sally Sue or Jess capillary electrophoresis-based protein analysis system (ProteinSimple, Santa Clara, CA, USA) was charged with protein lysates in equal amounts for each of Western blotting methods. As the antibody, antibodies to ALK, phospho-ALK, FGFR1, FGFR2, FGFR3, FGFR4, ERK, phospho-ERK, AKT, phospho-AKT, EGFR, phospho-EGFR, MET, β-actin, phospho-MET, HER2, CD44, BIM, VIM, CDH1, S6, pS6 and STAT3 (Cell Signaling Technology, Danvers, MA, USA), Cleaved PARP, phospho-HER2, FGF2 and FGF12 (Abcam) were used.

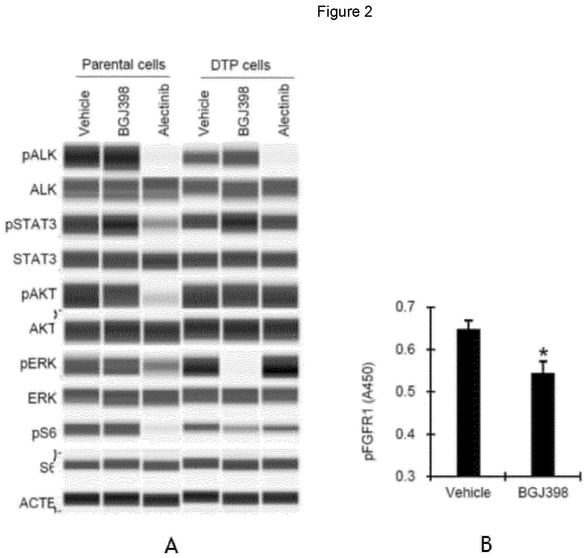

The results showed that the cell growth suppressive effects of treatment with PPP in parental cells and DTP cells were substantially equivalent (Figures 1A and B). For the alectinib treatment, an action of inhibiting the phosphorylation of ALK and the phosphorylation of ALK or STAT3, AKT, ERK and S6 as FGFR1 which are signal molecules was observed in parental cells, whereas an action of inhibiting the phosphorylation of STAT3, AKT, ERK and S6 was not observed in DTP cells (Figure 2A).

For the BGJ398 treatment, an action of inhibiting the phosphorylation of STAT3, AKT, ERK and ALK was not observed in parental cells, whereas an action of inhibiting the phosphorylation of FGFR1 and phosphorylation of ERK in DTP cells (Figures 2A and B).

Regrown cells prepared by culturing DTP cells in an alectinib-free medium for 37 days recovered sensitivity to alectinib and lost sensitivity to BGJ398 as compared to DTP cells (Figure 1C and Table 2).

The sensitivity of the regrown cells to these drugs was equivalent to that of the parental cells, and the DTP cells' biological characteristic of showing reversibility is similar to the characteristic of DTP cells from other cells which had been reported by Mikubo et al. [1].

A time-dependent change in ALK and FGFR1 signal molecules through alectinib treatment was analyzed for examining the mechanism of acquisition of dependency on FGFR1 signals in DTP cells. Among 23 types of FGF family members, 11 types of FGF ligands: FGFs-1, 2, 3, 4, 5, 6, 10, 19, 20, 21 and 22 are known to bind specifically to FGFR1 and activate signal molecules downstream of FGFR1 due to the activation of FGFR1 [2, 5]. The mRNA expression levels of these 11 types of FGFs in NCI-H2228 parental cells were analyzed, and the results showed that only the mRNA of FGF2 was expressed at 1 transcript per million (TPM) or more (Table 5).

**Table 5**

| B | FGFs | mRNA expression (log₂(TPM+1)) |
|---|---|---|
| | FGF1 | 0.19 |
| | FGF2 | 4.90 |
| | FGF3 | 0.00 |
| | FGF4 | 0.00 |
| | FGF5 | 0.03 |
| | FGF6 | 0.00 |
| | FGF10 | 0.06 |
| | FGF19 | 1.00 |
| | FGF20 | 0.00 |
| | FGF21 | 0.34 |
| | FGF22 | 0.10 |

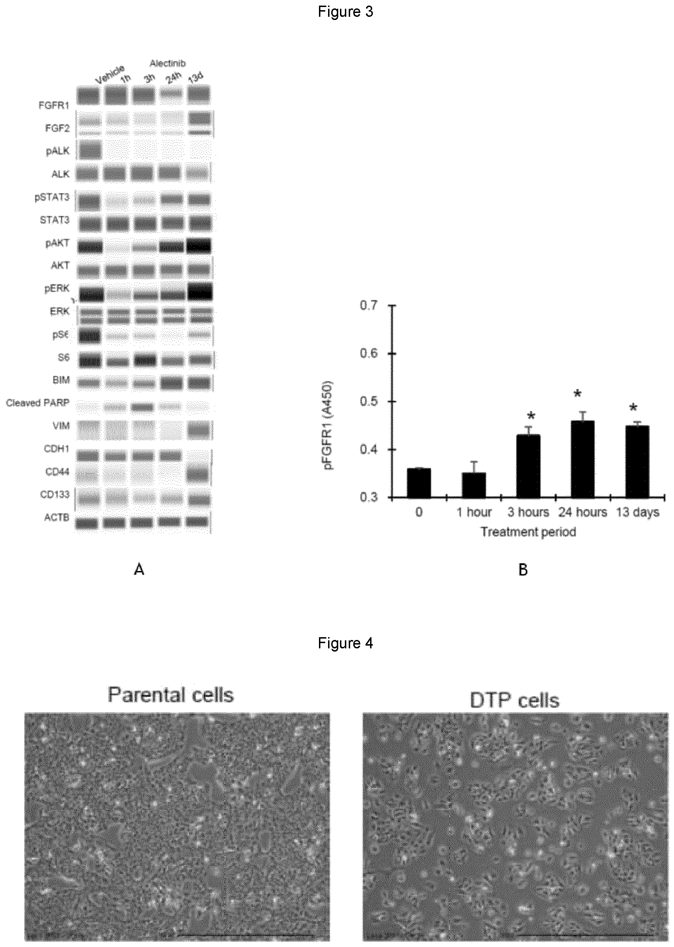

Thus, the expression levels of FGFR1 and FGF2 were examined. Thirteen days after the alectinib treatment, the amount of phosphorylation of FGFR1 and the amount of FGF2 protein increased (Figures 3A and B), whereas the amount of FGFR1 protein was substantially unchanged (Figure 3A).

Further, although the phosphorylation of ALK was thoroughly suppressed by alectinib treatment, the amount of phosphorylation of STAT3, AKT and ERK increased 13 days after the alectinib treatment (Figure 3A). DTP cells are known to exhibit a morphological change, stem cellularity, or epithelial-mesenchymal transformation (EMT) including increased vimentin (VIM) and decreased cadherin 1 (CDH1) [1, 6]. Even DTP cells with alectinib were shown to exhibit a morphological change (Figure 4), increased stem cell markers involving CD44 and CD133 proteins, and EMT associated with an increase in the amount of VIM protein and a decrease in the amount of CDH1 protein (Figure 3A).

### Example 2

For verifying whether activation of FGFR1 signals by an increase in expression of FGF2 protein contributes to the cell growth suppressive effect of treatment with an ALK inhibitor, gene-specific knockdown of FGFR1 and FGF2 was performed with small interfering RNA (siRNA) using NCI-H2228 cells.

### (1) Knockdown of FGFR1 or FGF2 with siRNA

Using NEPA21 Electroporation (Nepa Gene Co., Ltd., Japan), siRNA targeting FGFR1, FGF2 or a control (Horizon Discovery) was introduced into cells, and on the following day, a drug at a predetermined concentration was added, followed by culturing for 6 days. For knockdown of FGFR1 or FGF2, the results of examination by a Western blotting method after introduction of siRNA for 2 days showed that FGFR1 and FGF2 were eliminated (Figure 5A).

The FGFR1 or FGF2 knockout cells had increased sensitivity to alectinib as compared to control cells. The IC₄₀ values were 47.0 nM and 14.7 nM for FGFR1 knockout cells, 41.5 nM and 14.9 nM for FGF2 knockout cells, and > 1,000 nM and 246.4 nM for control cells (Figure 5B).

### (2) Establishment of FGFR1 and FGF2 knockout cells with CRISPR/Cas9 system

Using NEPA21 Electroporation, crRNA and Cas9 protein (Nippon Gene Co., Ltd.) targeting FGFR1 or FGF2 (Fasmac) were introduced into cells, two single clones were isolated from each clone cell with Smart Aliquotor (NT Science, Aichi, Japan), and it was confirmed by the Western blotting analysis that FGFR1 or FGF2 knockout cells lost FGFR1 or FGF2 protein (Figure 5C).

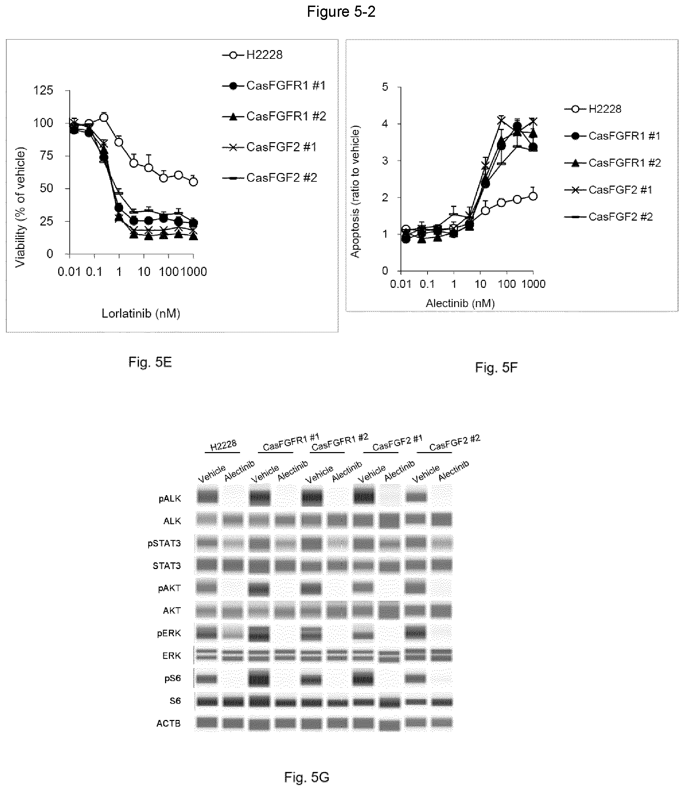

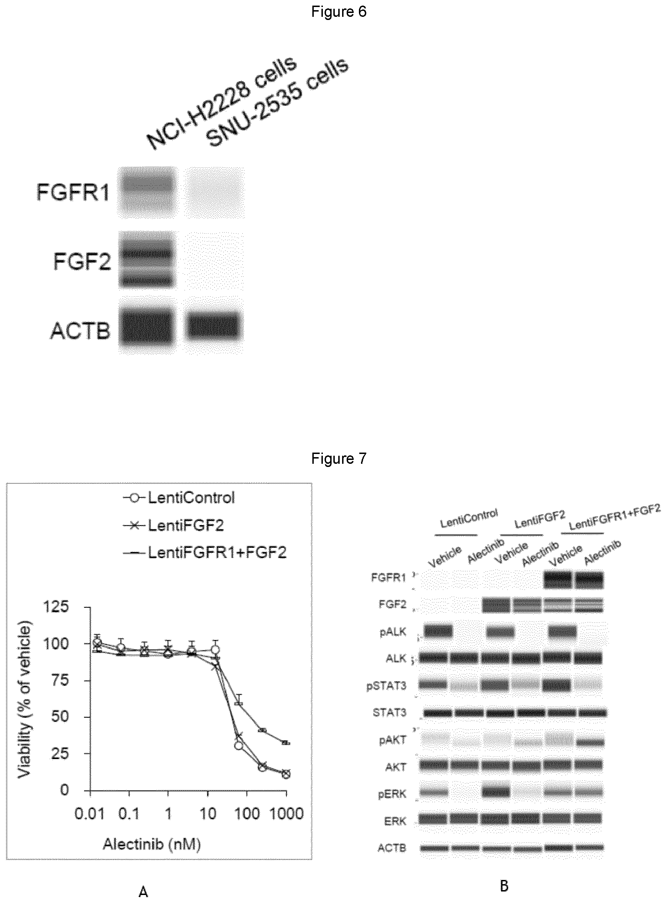

The FGFR1 or FGF2 knockout cells had increased sensitivity to alectinib as compared to parental cells. The IC₅₀ values were 5.4 nM and 7.3 nM for FGFR1 knockout cells, 5.6 nM and 6.1 nM for FGF2 knockout cells, and 219.6 nM for parental cells (Figure 5D). For the sensitivity under treatment with lorlatinib that is another ALK inhibitor, the IC₅₀ values were 0.7 nM and 0.7 nM for FGFR1 knockout cells, 0.7 nM and 0.9 nM for FGF2 knockout cells, and >1,000 nM for parental cells (Figure 5E).
The FGFR1 or FGF2 knockout cells obtained as described above were seeded on a 384-well plate, and on the following day, a drug was added at an indicated concentration, followed by culturing for an indicated time. The caspase 3 and 7 activity to signal induction of apoptosis in cells was measured using Caspase-Glo 3/7 Assay (Promega) in accordance with the protocol from the manufacturer. The activity was corrected for the cell viability determined in the manner described above, and subsequently, the relative caspase 3 and 7 activity by the drug against the vehicle was calculated. Each point represents an average + standard deviation from three experiments. The results showed that induction of inducible apoptosis (Figure 7B) and the action of inhibiting ERK (Figures 5F and 5G) by alectinib treatment were enhanced in the FGR1 or FGF2 knockout cells as compared to parental cells.

These findings suggest that cell survival against the ALK inhibitor is activated via FGFR1 signaling in ALK fusion gene-positive NSCLC cells with basal high expression of FGFR1 and FGF2, and protein molecules of both FGFR1 and FGF2 proteins are important for avoiding cell death resulting from suppression of cell growth by ALK inhibitor treatment in ALK fusion gene-positive NSCLC cells.

### Example 3

The expression levels of FGFR1 and FGF2 proteins were lower in SNU2535 parental cells than in NCI-H2228 parental cells (Figure 6).
For further confirming that both FGFR1 and FGF2 are necessary for cell survival in ALK inhibitor treatment, FGFR1 and FGF2 were excessively expressed in SNU2535 cells of ALK fusion gene-positive non-small cell lung cancer (NSCLC) (Figure 7B).

FGFR1, FGF2, or a human coding sequence (CDS) as a control was synthesized, and cloned downstream of an EF1A promoter in a lentivirus vector with VectorBuilder (Chicago, Illinois, USA), and SNU-2535 cells were infected with a lentivirus vector. After 1 day, the culture solution was cultured in a puromycin-containing medium for 4 days, cells excessively expressing FGFR1 and FGF2 were selected, and clones were isolated with Smart Aliquotor. The expression of FGFR1 or FGF2 protein was confirmed by the Western blotting analysis.

In the cells excessively expressing FGFR1 and FGF2, the cell growth suppressive effect of treatment with alectinib was decreased and the suppressive effect on the amount of phosphorylation of ERK was decreased as compared to control cells or cells excessively expressing FGF2 (Figures 7A and B).

These findings suggest that in ALK fusion gene-positive NSCLC cells with basal high expression of FGFR1 and FGF2, both FGFR1 and FGF2 proteins are expressed to activate FGFR1 signals, so that a cell growth suppressive action of ALK inhibitor treatment is avoided.

### Example 4

Using alectinib-resistant NCI-H2228 cells, whether FGFR1 signals were activated even after resistance to alectinib was examined.

NCI-H2228 cells were exposed to 1,000 nM alectinib for 348 days, and cultured in an alectinib-free medium for 60 days or more to establish resistant cells.

The cells were seeded on a 384-well plate, and treated with an ALK inhibitor (alectinib or lorlatinib (Selleck Chemicals LLC)) and a FGFR inhibitor (infigratinib (BGJ) (LC laboratories)) for 8 days, and a cell growth test was conducted as described Example 1.

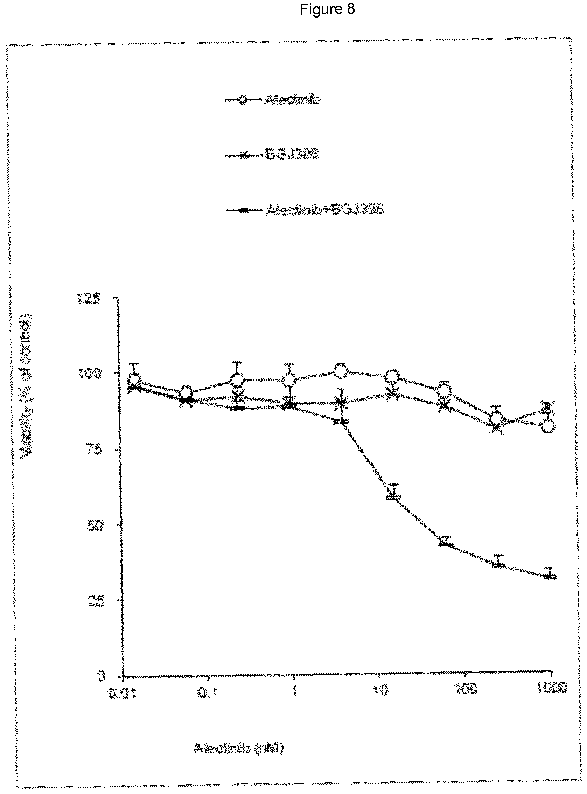

The results showed that the IC₅₀ value of alectinib was >1,000 nM for the resistant cells, and 219.6 nM for the parental cells. Thus, the IC₅₀ value for the resistant cells was equal to or greater than 4.6 times that for the parental cells (Table 6 and Figure 8).

**Table 6**

| A | Cells | | Alectinib (nM) | Alectinib+B GJ398 (nM) |
|---|---|---|---|---|
| | NCl-H2228 | Parental cells | 219.6 | 37.8 |
| | | Resistant cells | >1000 | 38.8 |

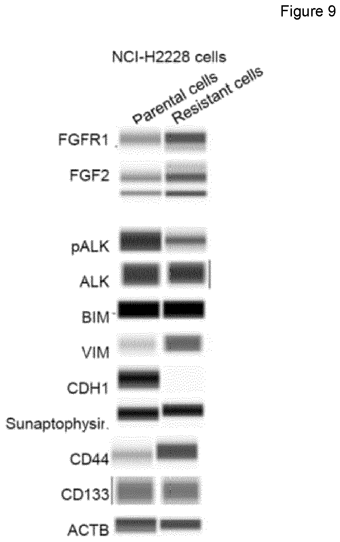

In the resistant cells, a resistance-associated mutation of ALK and an increase in the number of copies of FGFR1 and FGF2 genes did not occur, but an increase in the amounts of FGFR1 and FGF2 proteins was recognized (Table 7 and Figure 9).

**Table 7**

| Cells | | FGFR1 | FGF2 | ALK |
|---|---|---|---|---|
| NCI-H2228 | Parental cells | 2.1±0.0 | 1.7±0.2 | 1.7±0.2 |
| | Resistant cells | 2.3±0.1 | 1.8 ± 0.2 | 1.9±0.1 |

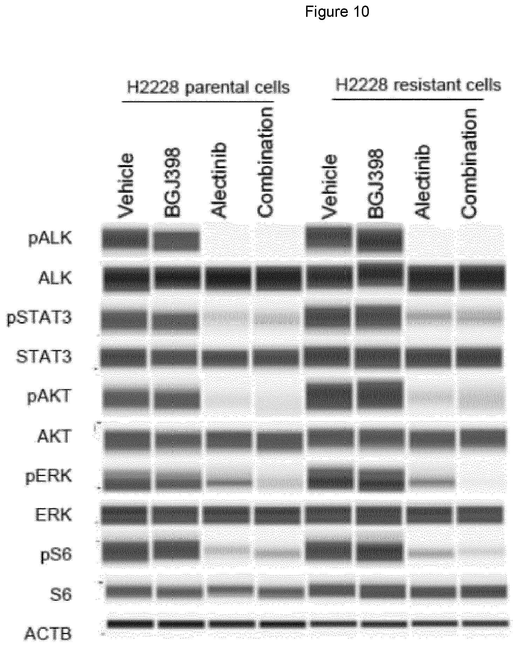

In the resistant cells, cell growth was strongly suppressed by treatment with BGJ398 and alectinib in combination, and the amount of phosphorylation of ERK decreased. The IC₅₀ value was >1,000 nM with alectinib alone, while being 38.8 nM in the combined use (Table 8 and Figure 10).

**Table 8**

| Cells | | Alectinib (nM) | BGJ398 (nM) | Alectinib+B GJ398 (nM) |
|---|---|---|---|---|
| NCl-H2228 | Parental cells | 219.6 | >1000 | 37.8 |
| | Resistant cells | >1000 | >1000 | 38.8 |

The above results suggest that in the resistant cells, the FGFR1 signals may be activated due to an increase in the amounts of the expression levels of FGFR1 and FGF2 proteins.

### Example 5

The effects of the combined use of the ALK inhibitor and the FGFR inhibitor in NCI-H2228 cells with basal high expression of FGFR1 and FGF2 and ALK fusion gene-positive SNU-2535 cells with basal low expression of FGFR1 and FGF2 were examined.

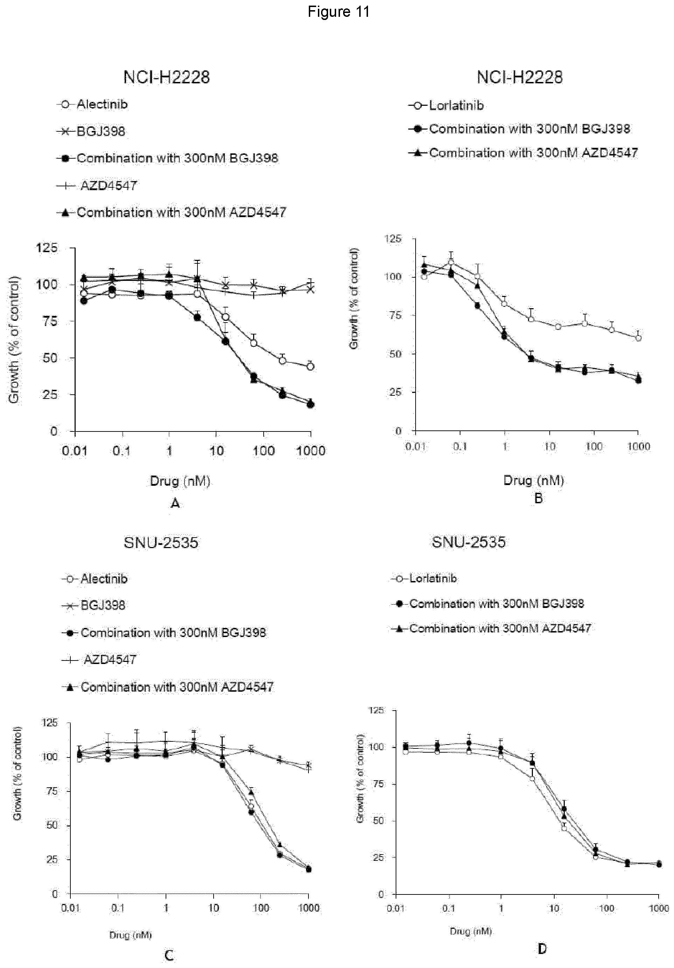

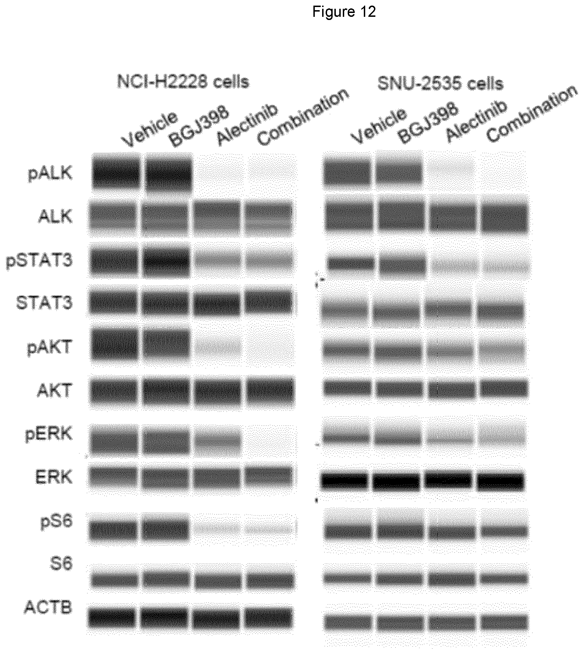

The results are shown in Figures 11 and 12. In Figure 11, the horizontal and vertical axes indicate the concentration of each drug and the cell viability, respectively.

In NCI-H2228 cells, sensitivity was not exhibited for the FGFR inhibitor alone (BGJ398 or AZD4547) (IC₅₀ value > 1,000 nM), but cell growth was suppressed by the combined use of the ALK inhibitor and the FGFR inhibitor (Figures 11A and B). Further, in NCI-H2228 cells, a suppressive action of combined use of alectinib and BGJ398 on phosphorylation of AKT and ERK was observed as compared to each single agent (Figure 12).

On the other hand, in SNU-2535 cells, an effect of the combined use of the ALK inhibitor and the FGFR inhibitor was not observed (Figures 11C and D).

Thus, it is shown that in the ALK fusion gene-positive NSCLC cells with basal high expression of FGFR1 and FGF2, FGFR1 signals are activated with the FGF2 binding to FGFR1, but the combined use of the ALK inhibitor and the FGFR inhibitor inhibits ALK and FGFR1 signals to exhibit a potent cell growth suppressive effect.

### Example 6

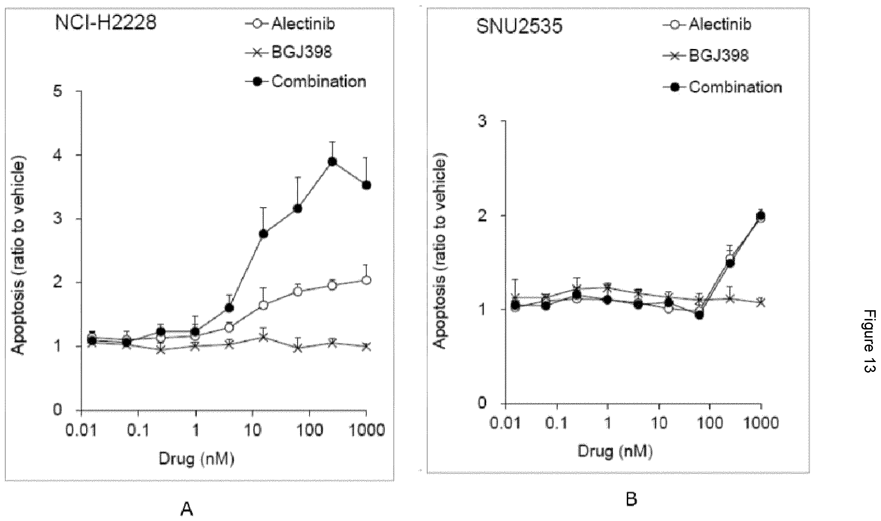

In HCI-H2228 cells, induction of apoptosis by alectinib treatment was enhanced by the use in combination with the FGFR inhibitor (Figure 13A). On the other hand, in SNU-2535 cells, an effect of the use in combination with the FGFR inhibitor (Figure 13B).

### Example 7

For evaluating the *in vivo* efficacy of combined treatment with the FGFR inhibitor and the ALK inhibitor, BGJ398 and alectinib were administered singly or in combination to nu/nu nude mice having a subcutaneous xenograft of NCI-H2228 cells.

From The Jackson Laboratory Japan, Inc. (Yokohama, Japan), 4 to 5-week-old male BALB/c-nu/nu mice (CAnN Cg-Foxn1<nu>/CrlCrlj nu/nu) were purchased.

Cells were subcutaneously inoculated to the right flank region at 5 × 10⁶ per mouse. The tumor volume and the body weight were measured twice weekly, where the tumor volume was estimated as below.

Tumor volume = ab²/2 (a and b indicate the length and the width of the tumor, respectively.

After the establishment of the tumor, the mice were assigned randomly to a solvent administration group, drug administration groups, or a combined administration group identical in dose and schedule to the group receiving each single drug, followed by oral administration for 11 days in a row.

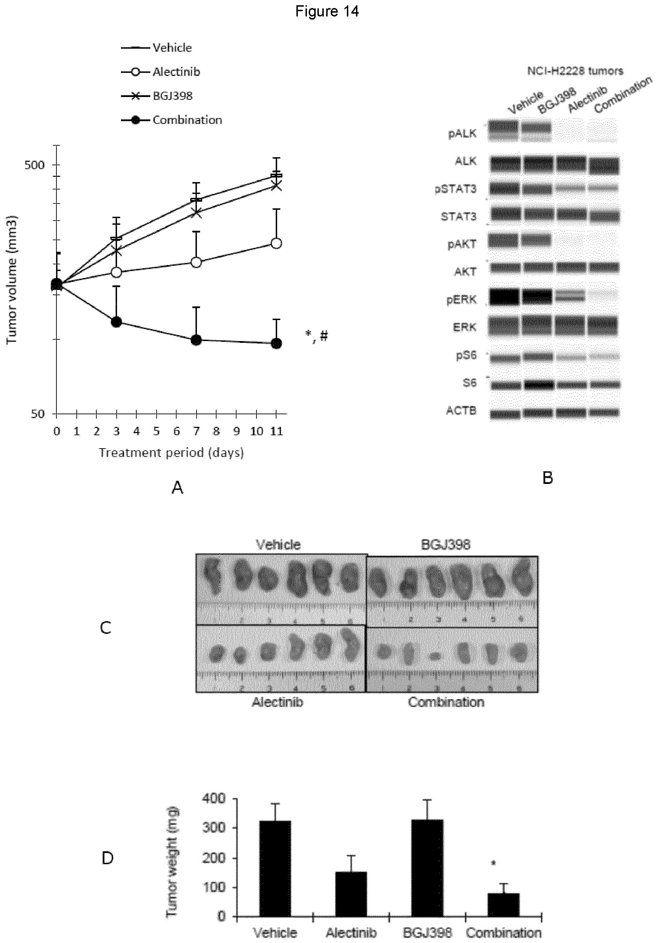

For the NCI-H2228 tumor, BGJ398 alone did not suppress tumor growth, but as compared to alectinib alone, the combined use had a significantly potent tumor growth suppressive effect (Figures 14A, C and D) and reduced the amount of phosphorylation of ERK (Figure 14B).

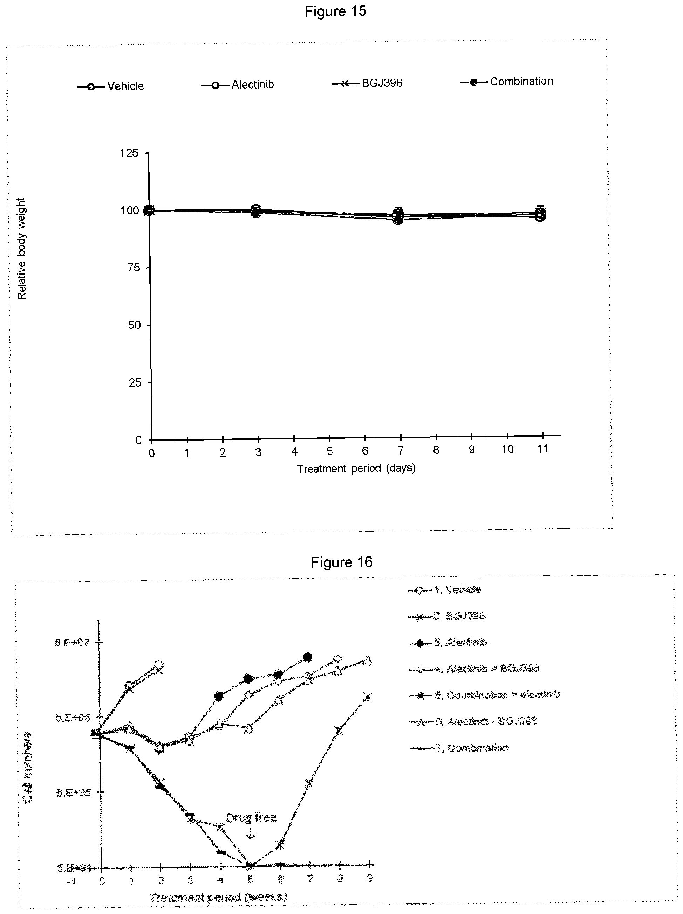

Further, the combined use was well tolerated, and did not cause a decrease in body weight during the treatment period (Figure 15).

An influence of long-term exposure to BGJ398 and alectinib singly or in combination in NCI-H2228 cells was evaluated *in vitro.* The treatment periods of the drugs are as in Table 9.

**Table 9**

| Treatment No. | 1st week | 2nd week | 3rd week | 4th week | 5th week | 6th week | 7th week | 8th week | 9th week |
|---|---|---|---|---|---|---|---|---|---|
| 1 | Solvent | | | | | | | | |
| 2 | BGJ398 | | | | | | | | |
| 3 | Alectinib | | | | | | | | |
| 4 | Alectinib | | BGJ398 | | | | | | |
| 5 | Combination | Alectinib | | | | No drug | | | |
| 6 | Alectinib | | BGJ398 | | Alectinib | | BGJ398 | | Alectinib |
| 7 | Combination | | | | | No drug | | | |

As shown in Figure 16, a potent cell growth suppressive effect was observed in the 5-week combination group (treatment No. 7) as compared to the groups receiving the drugs alternately (treatment Nos. 4 and 6). In the treatment No. 7, the cell growth suppressive effect up to the 5 weeks was substantially equivalent as compared to the group subjected to alectinib treatment for 4 weeks after combined use for only 1 week (treatment No. 5), and the growth suppressive effect continued even after replacement with a drug-free medium in the 6th week. In the treatment No. 5, the cells were regrown due to replacement with a drug-free medium in the 6th week (Figure 16).

The above results suggest that for strongly suppressing the cell growth of ALK fusion gene-positive NSCLC cells with basal high expression of FGFR1 and FGF2, it is useful to continue the combined use of the ALK inhibitor and the FGFR inhibitor for as long as possible.

### Example 8

The mRNA expression levels of FGFR1 and FGFR2 were measured using samples for the nonblinded ramdomized phase III test (J-ALEX clinical trial: JO28928) comparing the efficacy and the safety between crizotinib and alectinib targeting ALK fusion gene-positive progressive/recurrent non-small cell lung cancer, and associations with hazard ratios were analyzed.

Using the tumor samples collected before the start of treatment with alectinib (n = 38) and crizotinib (n = 31), the FGFR1 or FGF2 mRNA expression level was measured, various cutoff values were set (subset) for respective expression levels, and estimated hazard ratios (HRs) of alectinib and crizotinib were calculated. Using a multivariate Cox proportional hazard model, the adjusted hazard ratio (HR) and the 95% confidence band were estimated. In each subset, drugs considered to be related to progression of disease (PD) or death, ECOG performance status (PS) at the baseline, treatment and disease conditions at the start of treatment, and brain metastasis with IRF at the baseline were used as covariates for adjustment, and analysis was performed (Table 10).

**Table 10**

| A | | |
|---|---|---|
| | Covariate parameter | Variables |
| | FGFR1 of FGF2 mRNA | High/Low |
| | Treatment regimen | Alectinib / Crizotinib |
| | ECOG PS | 0/1 |
| | Treatment line | 1/2 |
| | Disease status | Stage IIB or IV / Postoperative reccurence |
| | Brain metastases at baseline by IRF | Yes/ No |

The distributions of PFS for specific cutoff values were estimated by the Kaplan-Meier method. The analyses were performed using R Studio version 1.0153 and R version 3.6.0. Table 11 shows the number of patients and the number of PFS events in each subset.

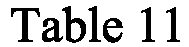

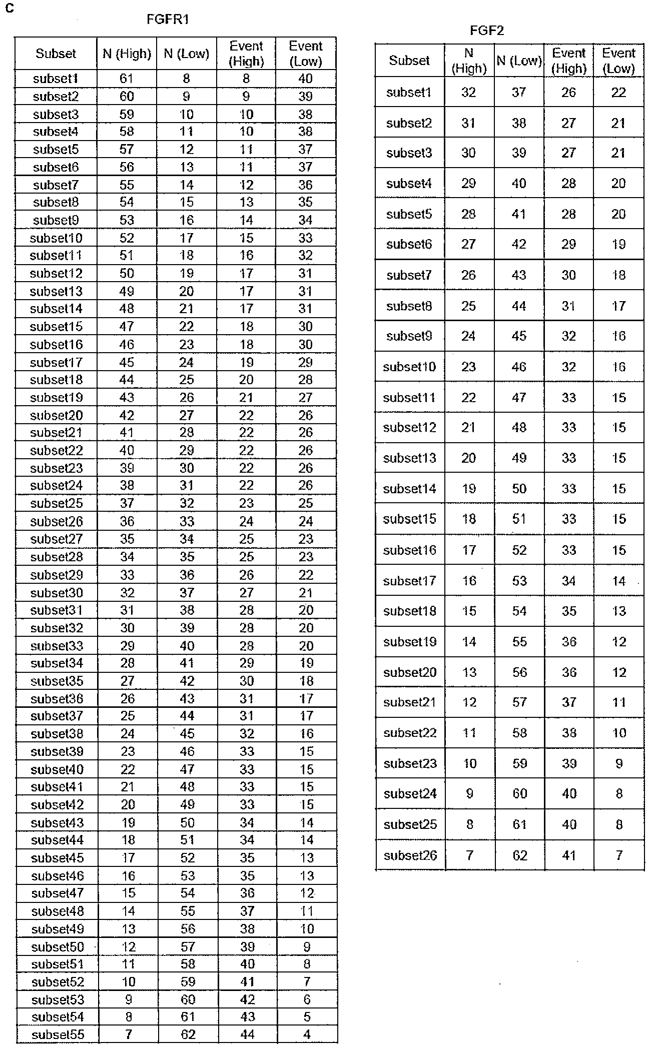

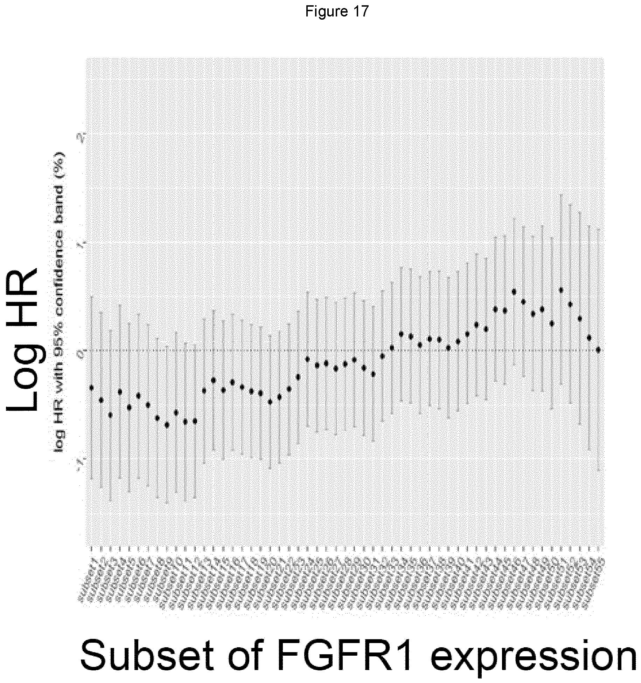

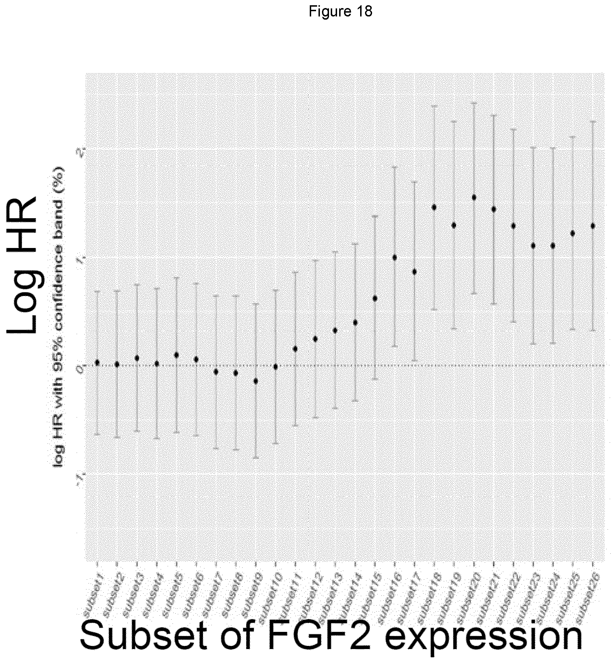

In Figures 17 and 18, the horizontal and vertical axes indicate the mRNA expression level and the hazard ratio respectively.

The results showed that the hazard ratios for the FGFR1 and FGF2 mRNA expression levels each tended to rise with an increase in mRNA level (Figures 17 and 18).

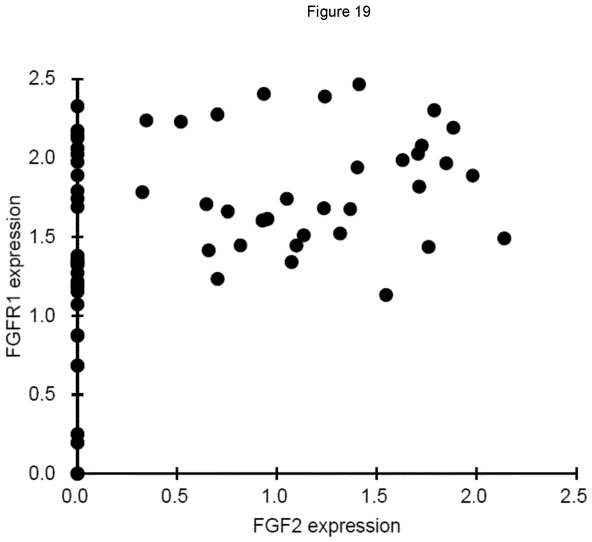

In particular, the cases of basal high expression of FGF2 mRNA which correspond to subsets 18 to 26 showed the highest hazard ratio with the log HR value being 1.0 or more (Figure 18). The patients with basal high expression of FGF2 mRNA underwent relatively high expression of FGFR1 mRNA as compared to the patients with basal low expression of FGF2 mRNA (Figure 19).
As above, the results of the clinical trial were consistent with those of the nonclinical trial in that in the patients with ALK fusion gene-positive NSCLC and basal high expression of FGFR1 mRNA and FGF2 mRNA, the efficacy of the ALK inhibitor was short in duration in primary treatment.

### Discussions

Currently, molecular-targeted therapies are standard therapies, and have a dramatic effect on many cancers having activated cancer genes, but these therapies hardly provide a complete cure. For ALK fusion gene-positive NSCLC, patients with complete responses account for less than 5% of patients receiving an ALK inhibitor [7]. In nonclinical trials, various DTP cells from molecular-targeted drug treatment have been identified, but the mechanism of induction of DTP has not been fully revealed [1].

According to the present application, DTP cells from alectinib treatment of cells with basal high expression of FGFR1 and FGF2 were shown to grow depending on FGFR signals. Further, the cell growth suppressive effect of the combined use of alectinib and the FGFR inhibitor was not observed in cells with basal low expression of FGFR1 and cells with basal low expression of FGF2, but was observed in cells with basal high expression of FGFR1 and cells with basal high expression of FGF2.

According to the present application, the results of analyzing data obtained in the clinical trial showed that in most of patients with an ALK mutation with basal high expression of FGF2, which account for about 20% of all the patients, the FGFR1 expression level was relatively high, and in these patients with basal high expression of FGF2, PFS was shorter than that in patients with basal low expression of FGF2.

Thus, the present application has revealed that in ALK fusion gene-positive cancer, basal high expression of both FGFR1 and FGF2 is involved in acquisition of resistance to the ALK inhibitor. Therefore, by performing a combined therapy with the ALK inhibitor and the FGFR inhibitor not after acquisition of resistance, but before acquisition of resistance, that is, in primary treatment in the case of clinical practice, potent treatment can be achieved which is capable of improving the prognosis of a lung cancer patient with ALK fusion gene-positive cancer without causing acquisition of resistance under the ALK inhibitor.

### (References)

1. Mikubo, M., et al., Mechanism of Drug Tolerant Persister Cancer Cells: The Landscape and Clinical Implication for Therapy.J Thorac Oncol, 2021. 16(11): p. 1798-1809.
2. Omitz, D.M. and N. Itoh, The Fibroblast Growth Factor signaling pathway. Wiley Interdiscip Rev Dev Biol, 2015. 4(3): p. 215-66.
3. Guagnano, V., et al., FGFR Genetic Alterations Predict for Sensitivity to NVP-BGJ398, a Selective Pan-FGFR Inhibitor.Cancer Discovery, 2012. 2(12): p. 1118-1133.
4. Menu, E., et al., Inhibiting the IGF-1 receptor tyrosine kinase with the cyclolignan PPP: an in vitro and in vivo study in the 5T33MM mouse model. Blood, 2006. 107(2): p. 655-60.
5. Tiong, K.H., L.Y. Mah, and C.-O. Leong, Functional roles of fibroblast growth factor receptors (FGFRs) signaling in human cancers.Apoptosis : an international journal on programmed cell death, 2013. 18(12): p. 1447-1468.
6. Hangauer, M.J., et al., Drug-tolerant persister cancer cells are vulnerable to GPX4 inhibition. Nature, 2017. 551(7679): p. 247-250.
30.Takezawa, K., et al., Role of ERK-BIM and STAT3-Survivin Signaling Pathways in ALK Inhibitor-Induced Apoptosis in EML4-ALK-Positive Lung Cancer. Clinical Cancer Research, 2011. 17(8): p. 2140-2148.
7. Camidge, D.R., et al., Updated Efficacy and Safety Data and Impact of the EML4-ALK Fusion Variant on the Efficacy of Alectinib in Untreated ALK-Positive Advanced Non-Small Cell Lung Cancer in the Global Phase III ALEX Study. Journal of Thoracic Oncology, 2019. 14(7): p. 1233-1243.

### [Industrial Applicability]

The combination drug of the present invention is useful for prevention or treatment ALK fusion gene-positive cancer positive for FGFR1 expression and positive for FGF2 expression at baseline, and the like. The combination drug of the present invention is useful as an agent for suppressing acquisition of resistance by ALK fusion gene-positive cancer positive for FGFR1 expression and positive for FGF2 expression at baseline. According to the present invention, it is possible to provide a method for selecting a patient with ALK fusion gene-positive cancer positive for FGFR1 expression and positive for FGF2 expression at baseline.

## Claims

1. A drug for treating or preventing ALK fusion gene-positive cancer positive for FGFR1 expression and positive for FGF2 expression at baseline, the drug comprising an ALK inhibitor and a FGFR inhibitor in combination.

2. A drug for suppressing the acquisition of resistance by ALK fusion gene-positive cancer positive for FGFR1 expression and positive for FGF2 expression at baseline, the drug comprising an ALK inhibitor and a FGFR inhibitor in combination.

3. A drug for treating or preventing ALK fusion gene-positive cancer positive for FGFR1 expression and positive for FGF2 expression at baseline, the drug comprising an ALK inhibitor as an active ingredient, which is used in combination with a FGFR inhibitor.

4. A drug for suppressing the acquisition of resistance by ALK fusion gene-positive cancer positive for FGFR1 expression and positive for FGF2 expression at baseline, the drug comprising an ALK inhibitor as an active ingredient, which is used in combination with a FGFR inhibitor.

5. A drug for treating or preventing ALK fusion gene-positive cancer positive for FGFR1 expression and positive for FGF2 expression at baseline, the drug comprising a FGFR inhibitor as an active ingredient, which is used in combination with an ALK inhibitor.

6. A drug for suppressing the acquisition of resistance by ALK fusion gene-positive cancer positive for FGFR1 expression and positive for FGF2 expression at baseline, the drug comprising a FGFR inhibitor as an active ingredient, which is used in combination with an ALK inhibitor.

7. The drug according to any one of claims 1 to 6, wherein the ALK inhibitor is a compound selected from crizotinib, alectinib, ceritinib, lorlatinib and brigatinib, or a salt thereof.

8. The drug according to any one of claims 1 to 6, wherein the FGFR inhibitor is a compound selected from the group consisting of infigratinib, derazantinib, pemigatinib, erdafitinib, ponatinib, Debio 1347, futibatinib, tasurgratinib, AZD 4547 and PD 173074, or a salt thereof.

9. The drug according to any one of claims 1 to 6, wherein the ALK fusion gene-positive cancer is cancer selected from the group consisting of non-Hodgkin's lymphoma, neuroblastoma, inflammatory myofibroblastic cytoma, kidney cancer and lung cancer.

10. The drug according to any one of claims 1 to 6, wherein the ALK fusion gene-positive cancer has not received prior treatment with an ALK inhibitor.

11. The drug according to any one of claims 1 to 6, wherein the ALK fusion gene-positive cancer is sensitive to ALK.

12. The drug according to any one of claims 1 to 6, wherein the ALK inhibitor is administered simultaneously with the FGFR inhibitor.

13. A method for selecting a patient with ALK fusion gene-positive cancer positive for FGFR1 expression and positive for FGF2 expression at baseline, the method comprising acquiring a tumor tissue of a patient diagnosed as ALK fusion gene-positive cancer, and measuring expression levels of FGFR1 and FGF2 in the tumor tissue specimen.
